# EUROPEAN PATENT APPLICATION

(11) **EP 4 647 476 A1**
(43) Date of publication of application: **12.11.2025**
(21) Application number: 25174702.8
(22) Date of filing: 07.05.2025
(51) Int. Cl.: C09K 11/06, C07D 487/04, H10K 50/11, H10K 85/60

(54) **ORGANIC COMPOUND AND ORGANIC LIGHT-EMITTING ELEMENT**

(30) Priority: 09.05.2024 JP 2024076481
(71) Applicant: CANON KABUSHIKI KAISHA, Tokyo 146-8501 (JP)
(72) Inventor: KIKUCHI, Takayoshi, Tokyo, 146-8501 (JP); NISHIDE, Yosuke, Tokyo, 146-8501 (JP); YAMADA, Naoki, Tokyo, 146-8501 (JP)
(74) Representative: TBK

(57) **Abstract**

An organic compound represented by formula (1):

(A)ₙ₁-L-(B)ₙ₂ (1)

in formula (1), (A) has a structure represented by formula (2), and (B) has a structure represented by formula (3) or formula (4): 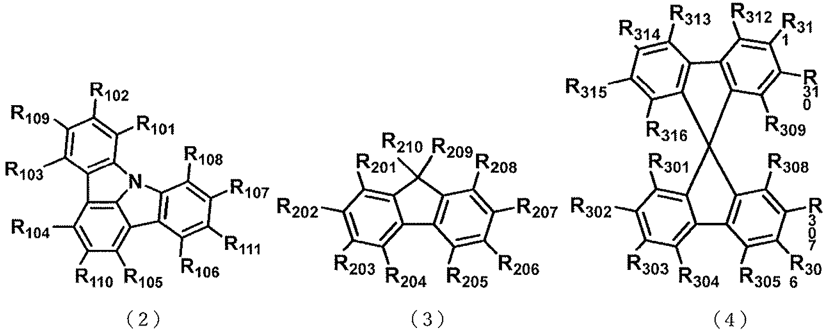 in formulae (2) to (4), R₁₀₁ to R₃₁₆ are each a hydrogen atom or any substituent; L is a linking group; one of R₁₀₁ to R₁₁₁ in formula (2), one of R₂₀₁ to R₂₀₈ in formula (3), and one of R₃₀₁ to R₃₀₈ in formula (4) are the binding positions to L.

## Description

### BACKGROUND

### Technical Field

The present disclosure relates to an organic compound and an organic light-emitting element using it.

### Description of the Related Art

An organic light-emitting element (which may be referred to as an "organic electroluminescent element" or an "organic EL element") is an electron element including a pair of electrodes and an organic compound layer disposed between the electrodes. An exciton of a light-emitting organic compound in the organic compound layer is generated by injection of an electron and a hole from the pair of electrodes, and the organic light-emitting element emits light when the exciton returns to the ground state.

A recent progress of the organic light-emitting elements is remarkable, and the characteristics of the progress are, for example, a low drive voltage, a wide variety of emission wavelengths, high-speed response, and ability to make light emitting devices thinner and lighter.

In addition, compounds suitable for organic light-emitting elements have been actively created up to now, which is because creation of compounds with excellent element life characteristics is important for providing high-performance organic light-emitting elements. U.S. Patent Application Publication No. 2016/0260908 describes Compound 1-a as a compound that has been created.

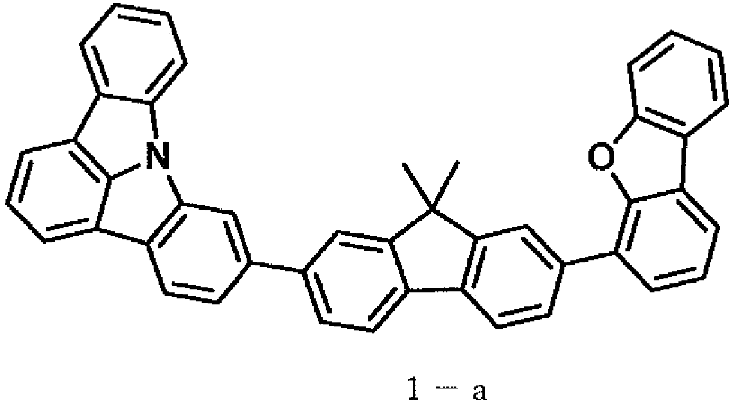

However, since the Compound 1-a is a 2-substituted fluorene which is a fluorene having indolocarbazole and dibenzofuran as substituents, the lowest excited triplet (T1) energy value is low.

### SUMMARY

The present disclosure has been made in view of the above disadvantages and provides an organic compound exhibiting a high T1 energy value.

The present disclosure in its first aspect provides an organic compound as specified in claims 1 to 12.

The present disclosure in its second aspect provides an organic light-emitting element as specified in claims 13 to 16, and the present disclosure in its third to ninth aspects provides a display apparatus, a photoelectric conversion apparatus, an image display apparatus, electronic equipment, a wearable device, an illuminating apparatus, and a moving object as specified in claims 17 to 23, respectively.

Further features of the present disclosure will become apparent from the following description of exemplary embodiments with reference to the attached drawings.

### BRIEF DESCRIPTION OF THE DRAWINGS

Fig. 1A is a schematic cross-sectional view illustrating an example of a pixel of a display apparatus according to an embodiment of the present disclosure. Fig. 1B is a schematic cross-sectional view of an example of a display apparatus using an organic light-emitting element according to an embodiment of the present disclosure.
Fig. 2 is a schematic view illustrating an example of a display apparatus according to an embodiment of the present disclosure.
Fig. 3A is a schematic view illustrating an example of an image pickup apparatus according to an embodiment of the present disclosure. Fig. 3B is a schematic view illustrating an example of electronic equipment according to an embodiment of the present disclosure.
Fig. 4A is a schematic view illustrating an example of a display apparatus according to an embodiment of the present disclosure. Fig. 4B is a schematic view illustrating an example of a foldable display apparatus.
Fig. 5A is a schematic view illustrating an example of an illuminating apparatus according to an embodiment of the present disclosure. Fig. 5B is a schematic view illustrating an example of an automobile having a vehicle lighting device according to an embodiment of the present disclosure.
Fig. 6A is a schematic view illustrating an example of a wearable device according to an embodiment of the present disclosure. Fig. 6B is a schematic view illustrating an example of a wearable device in the form of having an image pickup apparatus according to an embodiment of the present disclosure.
Fig. 7A is a schematic view of an image formation apparatus according to an embodiment of the present disclosure. Figs. 7B and 7C are schematic views illustrating configurations in which a plurality of light-emitting units of an exposure light source are disposed on a long substrate.

### DESCRIPTION OF THE EMBODIMENTS

In the present specification, examples of the halogen atom include, but not limited to, a fluorine atom, a chlorine atom, a bromine atom, an iodine atom, an astatine atom, and a tennessine atom.

The alkyl group may be an alkyl group having 1 or more and 20 or less carbon atoms or may be an alkyl group having 1 or more and 10 or less carbon atoms. Specifically, examples thereof include, but not limited to, a methyl group, an ethyl group, a normal propyl group, an isopropyl group, a normal butyl group, a tertiary butyl group, a secondary butyl group, an octyl group, a cyclohexyl group, a tertiary pentyl group, a 3-methylpentan-3-yl group, a 1-adamantyl group, and a 2-adamantyl group.

The alkoxy group may an alkoxy group having 1 or more and 20 or less carbon atoms or may be an alkoxy group having 1 or more and 10 or less carbon atoms. Specifically, examples thereof include, but not limited to, a methoxy group, an ethoxy group, a propoxy group, an isopropoxy group, a tertiary butoxy group, a 2-ethyl-octyloxy group, and a benzyloxy group.

Specifically, examples of the silyl group include, but not limited to, a trimethylsilyl group and a triphenylsilyl group.

The aryl group may have 6 or more and 20 or less carbon atoms, 6 or more and 18 or less carbon atoms, or 6 or more and 12 or less carbon atoms. Specifically, examples thereof include, but not limited to, a phenyl group, a biphenyl group, a naphthyl group, a phenanthrenyl group, a triphenyrenyl group, an indenyl group, a terphenyl group, a fluorenyl group, a pyrenyl group, an anthranil group, a perylenyl group, a chrysenyl group, and a fluoranthenyl group.

The heterocyclic group may have 3 or more and 24 or less carbon atoms, 3 or more and 18 or less carbon atoms, or 3 or more and 12 or less carbon atoms. Specifically, examples thereof include, but not limited to, a pyridyl group, a pyrimidyl group, a pyrazyl group, a triazyl group, a benzofuranyl group, a benzothiophenyl group, a dibenzofuranyl group, a benzothiophenyl group, an oxazolyl group, an oxadiazolyl group, a thiazolyl group, a thiadiazolyl group, a carbazolyl group, an acridinyl group, and a phenanthrolyl group.

The amino group may be a substituted amino group substituted with an alkyl group or an aryl group and may be a substituted amino group substituted with an alkyl group having 1 or more and 4 or less carbon atoms or an aryl group having 6 or more and 12 or less carbon atoms. Specifically, examples thereof include, but not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-tertiary butylphenyl)amino group, an N-phenyl-N-(4-trifluoromethylphenyl)amino group, and an N-piperidyl group.

Specifically, examples of the aryloxy group include, but not limited to, a phenoxy group.

Specifically, examples of the heteroaryloxy group include, but not limited to, a thienyloxy group.

The above-mentioned alkyl group, alkoxy group, amino group, aryloxy group, silyl group, aryl group, heterocyclic group, and heteroaryloxy group may further have substituents, and examples of the substituents include, but not limited to, deuterium; alkyl groups such as a methyl group, an ethyl group, a normal propyl group, an isopropyl group, a normal butyl group, and a tertiary butyl group; aralkyl groups such as a benzyl group; aryl groups such as a phenyl group and a biphenyl group; heterocyclic groups such as a pyridyl group and a pyrrolyl group; amino groups such as a dimethylamino group, a diethylamino group, a dibenzylamino group, a diphenylamino group, and a ditolylamino group; alkoxy groups such as a methoxy group, an ethoxy group, and a propoxy group; aryloxy groups such as a phenoxy group; halogen atoms such as fluorine, chlorine, bromine, and iodine; and a cyano group.

### [1] Organic compound

An organic compound according to the present embodiment will now be described. The organic compound of the present embodiment is represented by the following general formula (1):

(A)ₙ₁-L-(B)ₙ₂ (1).

In the general formula (1), (A) has a structure represented by a general formula (2), and (B) has a structure represented by a general formula (3) or (4).

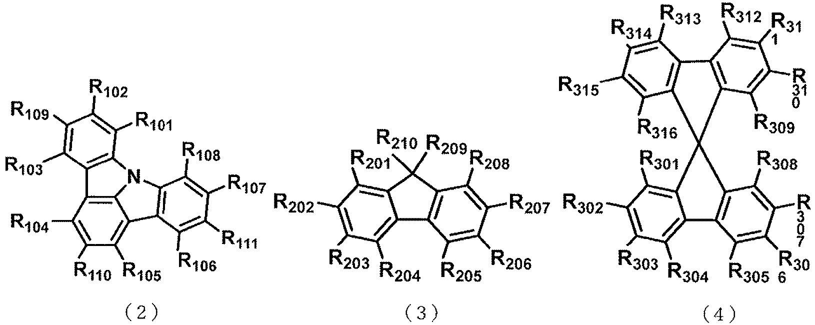

In the general formula (2), R₁₀₁ to R₁₁₁ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group. Any one of R₁₀₁ to R₁₁₁ is a binding position to L.

Hereinafter, the general formula (2) may be referred to as an indolocarbazole skeleton, the general formula (3) may be referred to as a fluorene skeleton, and the general formula (4) may be referred to as a fluorene skeleton or a spirofluorene skeleton.

In the general formula (2), any one of R₁₀₁ to R₁₀₈ can bind to L; any one of R₁₀₂, R₁₀₃, R₁₀₄, R₁₀₅, R₁₀₆, or R₁₀₇ can bind to L; or R₁₀₂ or R₁₀₇ can bind to L. When the indolocarbazole skeleton binds to L at these positions, T1 shows a higher value.

In the general formula (2), R₁₀₁ to R₁₁₁ can be each independently selected from the group consisting of a hydrogen atom, a deuterium atom, a fluorine atom, an alkyl group having 1 or more and 4 or less carbon atoms, an alkoxy group having 1 or more and 4 or less carbon atoms, and a trimethylsilyl group.

In the general formula (3), R₂₀₁ to R₂₀₈ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group. R₂₀₉ and R₂₁₀ are each independently selected from the group consisting of a hydrogen atom, a deuterium atom, a halogen atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, and a cyano group. Any one of R₂₀₁ to R₂₀₈ is a binding position to L.

In the general formula (3), any one of R₂₀₂, R₂₀₃, R₂₀₄, R₂₀₅, R₂₀₆, or R₂₀₇ can bind to L, or R₂₀₃ or R₂₀₆ can bind to L.

In the general formula (3), R₂₀₁ to R₂₀₈ can be each independently selected from the group consisting of a hydrogen atom, a deuterium atom, a fluorine atom, an alkyl group having 1 or more and 6 or less carbon atoms, an alkoxy group having 1 or more and 4 or less carbon atoms, and a trimethylsilyl group; and R₂₀₉ and R₂₁₀ can be each independently selected from the group consisting of a hydrogen atom, a deuterium atom, an alkyl group having 1 or more and 4 or less carbon atoms, and an aryl group having 6 or more and 12 or less carbon atoms. R₂₀₁ to R₂₀₈ may be hydrogen atoms, and R₂₀₉ and R₂₁₀ may be methyl groups.

In the general formula (4), R₃₀₁ to R₃₀₈ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group. Any one of R₃₀₁ to R₃₀₈ is a binding position to L. R₃₀₉ to R₃₁₆ are each independently selected from the group consisting of a hydrogen atom, a deuterium atom, a halogen atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, and a cyano group.

In the general formula (4), any one of R₃₀₂, R₃₀₃, R₃₀₄, R₃₀₅, R₃₀₆, or R₃₀₇ can bind to L, and R₃₀₃ or R₃₀₆ can bind to L.

In the general formula (4), R₃₀₁ to R₃₁₆ can be each independently selected from the group consisting of a hydrogen atom, a deuterium atom, a fluorine atom, an alkyl group having 1 or more and 6 or less carbon atoms, an alkoxy group having 1 or more and 4 or less carbon atoms, and a trimethylsilyl group; and R₃₀₁ to R₃₁₆ may be each independently selected from the group consisting of a hydrogen atom and an alkyl group having 1 or more and 4 or less carbon atoms.

In the general formula (1), L is a linking group derived from a substituted or unsubstituted phenyl group, a linking group derived from a substituted or unsubstituted naphthyl group, a combination thereof, or a direct bond.

When L includes at least one of a phenyl group having a substituent or a naphthyl group having a substituent, the substituent is not specifically limited, but may be, for example, an alkyl group having 1 or more and 6 or less carbon atoms or an aryl group having 6 or more and 18 or less carbon atoms, may be an alkyl group having 1 or more and 4 or less carbon atoms or an aryl group having 6 or more and 10 or less carbon atoms, or may be a methyl group or a phenyl group. When L has a phenyl group, the phenyl group may be a metaphenylene group or metabiphenylene group having a binding position at the meta-position or a combination thereof.

In the general formula (1), n1 and n2 are each an integer of 1 or more and 3 or less. When n1 is 2 or more, (A)s may have the same or different structures. When n2 is 2 or more, (B)s may have the same or different structures. From the viewpoint of sublimability, n1 can be 1, and n2 can be 1 or 2.

Furthermore, the organic compound according to the present embodiment can be a compound represented by any one of the general formulae (5) to (8):

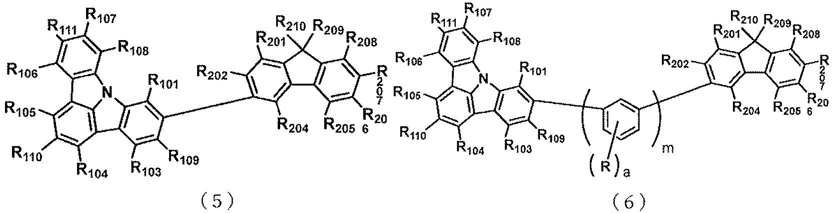

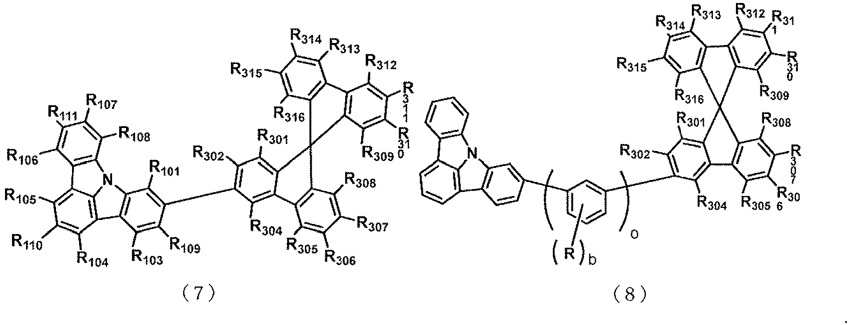

In the general formula (6), R is a deuterium atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, or a cyano group. When the phenyl group as the linking group has R, R may be an alkyl group having 1 or more and 4 or less carbon atoms or an aryl group having 6 or more and 10 or less carbon atoms or may be a methyl group or a phenyl group.

In the general formula (6), "a" is an integer of 0 or more and 4 or less, and "m" is an integer of 1 or more and 4 or less. When there is a plurality of Rs, Rs may be the same or different.

In the general formula (8), R is a deuterium atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, or a cyano group. When the phenyl group as the linking group has R, R may be an alkyl group having 1 or more and 4 or less carbon atoms or an aryl group having 6 or more and 10 or less carbon atoms or may be a methyl group or a phenyl group.

In the general formula (8), "b" is an integer of 0 or more and 4 or less, and "o" is an integer of 1 or more and 4 or less. When there is a plurality of Rs, Rs may be the same or different.

The organic compound according to the present embodiment will now be described in detail.

The organic compound according to the present embodiment has the following characteristics:
(1-1) In the general formulae (3) and (4), R₂₀₁ to R₂₀₈ and R₃₀₁ to R₃₁₆ do not have an aryl group and a heterocyclic group other than L (when L is a direct bond, the general formula (2)); and
(1-2) In the general formula (1), B is a fluorene skeleton or a spirofluorene skeleton.

These characteristics will be described below.

(1-1) In the general formulae (3) and (4), R₂₀₁ to R₂₀₈ and R₃₀₁ to R₃₁₆ do not have an aryl group and a heterocyclic group other than L (when L is a direct bond, the general formula (2)).

As a result of extensive investigations, the present inventors have found that when in the general formulae (3) and (4), R₂₀₁ to R₂₀₈ and R₃₀₁ to R₃₁₆ do not have an aryl group and a heterocyclic group other than L (when L is a direct bond, the general formula (2)), an organic compound with a high T1 energy is obtained.

Effects by using an organic compound with a high T1 energy will now be described.

A compound in the organic compound layer, in particular, the light-emitting layer, of an organic light-emitting element undergoes repeated transitions between the ground state and the excited state in the process of light emission of the organic light-emitting element. In particular, in an organic light-emitting element including a phosphorescent dopant (also called phosphorescent guest) as the light-emitting dopant (called light-emitting guest or a guest material), it is important to control T1 excitons, which account for 75% of excited states.

The organic light-emitting element including a phosphorescent dopant is an organic light-emitting element that uses a T1 energy for light emission. The host material and peripheral materials in the light-emitting layer of the organic light-emitting element can have a sufficiently higher T1 energy than the phosphorescent dopant that emits phosphorescence.

When the host material has a sufficiently higher T1 energy than the phosphorescent dopant, energy transfer from the host material to the phosphorescent dopant is accelerated. Accordingly, since the time that the host material is in the unstable triplet state is shortened, degradation of the host material can be suppressed. In addition, since the probability of using the generated excitation energy in the reaction with adjacent molecules becomes low, generation of quencher molecules can be decreased. As a result, since the energy transfer from the host material to the phosphorescent dopant is accelerated, the organic light-emitting element using a host material having a sufficiently high T1 energy can exhibit excellent light emission efficiency. In addition, since the energy transfer from the host material to the phosphorescent dopant can be accelerated, an excellent element life can be obtained.

Specifically, when an organic light-emitting element emits green phosphorescence, the T1 energy of the host material can be 0.1 eV or more higher than 2.43 eV (wavelength conversion: 510 nm). When an organic light-emitting element emits red phosphorescence, the T1 energy of the host material can be 0.1 eV or more higher than 2.07 eV (wavelength conversion: 600 nm).

In the organic compound according to the present embodiment, when R₂₀₁ to R₂₀₈ and R₃₀₁ to R₃₁₆ in the general formulae (3) and (4) do not include an aryl group and a heterocyclic group other than the general formula (2) and L, the spread of conjugation can be suppressed. As a result, the organic compound according to the present embodiment can have a high T1 energy.

Table 1 shows T1 wavelengths calculated from the T1 energies determined by molecular orbital calculation of Exemplary Compound A10, which is an organic compound according to the present embodiment, and Comparative Compound 1-a.

**Table 1**

| | Molecular structure | T1 (nm) |
|---|---|---|
| Exemplary Compound A10 | 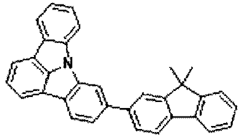 | 464 |
| Comparative Compound 1-a | 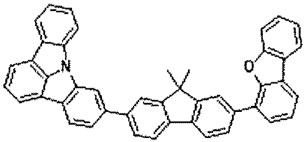 | 481 |

As shown in Table 1, Exemplary Compound A10 exhibited a shorter T1 wavelength compared to Comparative Compound 1-a. Since the shorter the wavelength, the higher the energy, Exemplary Compound A10 exhibits a higher T1 energy than Comparative Compound 1-a. This difference is caused by the type and number of the substituent possessed by the fluorene skeleton. Specifically, although the fluorene skeleton represented by the general formula (3) of Exemplary Compound A10 binds to the indolocarbazole skeleton represented by the general formula (2), the fluorene skeleton represented by the general formula (3) of Comparative Compound 1-a binds to the indolocarbazole skeleton represented by the general formula (2) and a benzofuran skeleton. It is inferred that since the fluorene skeleton of Comparative Compound 1-a includes an indolocarbazole skeleton and a benzofuran skeleton, the spread of conjugation is increased, and Comparative Compound 1-a exhibits a longer T1 wavelength than the T1 wavelength of Exemplary Compound A10.

In contrast, since the fluorene skeleton of the organic compound according to the present embodiment does not have an aryl group, a heterocyclic group, or substituents derived therefrom, other than the indolocarbazole skeleton. As a result, since the spread of conjugation can be suppressed, it is inferred that a short T1 wavelength (high T1 energy) is exhibited.

In the organic compound according to the present embodiment, the fluorene skeleton represented by the general formula (3) may include an aryl group, a heterocyclic group, or a substituent derived therefrom as long as the position thereof is R₂₀₉ or R₂₁₀. It is because that the position 9 and the position 9' (corresponding to R₂₀₉ and R₂₁₀) of the fluorene skeleton are binding positions where conjugation is difficult to spread (conjugation is broken), and the T1 energy is therefore less influenced.

In the organic compound according to the present embodiment, when any one of R₃₀₁ to R₃₀₈ of the spirofluorene skeleton represented by the general formula (4) binds to L, R₃₀₉ to R₃₁₆ may include an aryl group, a heterocyclic group, or a substituent derived therefrom. Since the spirofluorene skeleton is a skeleton formed by condensation of the substitutions at the position 9 and position 9' of the fluorene skeleton, even if R₃₀₉ to R₃₁₅ have an aryl group, a heterocyclic group, or a substituent derived therefrom, conjugation is difficult to spread (conjugation is broken), and the T1 energy is therefore less influenced.

Table 2 shows the T1 wavelengths when the position 9 or position 9' of the T1 fluorene skeleton includes and does not include an aryl group.

**Table 2**

| | Embodiment A | Embodiment B |
|---|---|---|
| Structural formula | 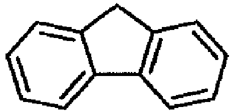 | 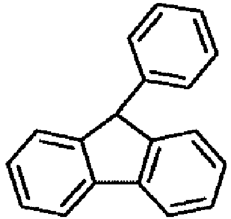 |
| T1 wavelength (nm) | 401 | 403 |

As shown in Table 2, the embodiment A of which the fluorene skeleton does not include an aryl group at the position 9 or position 9' has a T1 wavelength of 401 nm, the embodiment B of which the fluorene skeleton includes an aryl group at the position 9 or position 9' has a T1 wavelength of 403 nm, and it is demonstrated that both exhibit similar T1 wavelengths. Based on this result, in the organic compound according to the present embodiment, R₂₀₉ and R₂₁₀ may include an aryl group, a heterocyclic group, or a substituent derived therefrom.

As described above, the organic compound according to the present embodiment can exhibit a high T1 energy by that R₂₀₁ to R₂₀₈ and R₃₀₁ to R₃₁₆ in the general formulae (3) and (4) do not include an aryl group and a heterocyclic group other than the general formula (2) and L.

(1-2) In the general formula (1), B is a fluorene skeleton or a spirofluorene skeleton.

The organic compound according to the present embodiment has a small polarity as a whole molecule by that the skeleton represented by the general formulae (3) or (4) is a fluorene skeleton or a spirofluorene skeleton. As a result, since the interaction between the organic compound molecules according to the present embodiment can be reduced, the organic compound according to the present embodiment hardly aggregates. In other words, compatibility between the organic compound according to the present embodiment and the phosphorescent dopant is increased.

Since the organic compound according to the present embodiment has high compatibility with the phosphorescent dopant, the distance between the organic compound according to the present embodiment and the phosphorescent dopant becomes closer. It is known that the energy transfer process from the organic compound according to the present embodiment to the guest material is caused by dexter energy transfer. Accordingly, the intermolecular distance between the organic compound according to the present embodiment and the phosphorescent dopant becomes closer, and thereby the energy transfer is more accelerated. As a result, an organic light-emitting element using the organic compound according to the present embodiment has excellent light emission efficiency. In addition, an organic light-emitting element using the organic compound according to the present embodiment has an excellent element life.

Table 3 shows comparison between Exemplary Compound A2 which is an organic compound according to the present embodiment and Comparative Compound 1-b which is a comparative example. Light emission efficiency and Element life in Table 3 are respectively relative values when the light emission efficiency and the element life of an organic light-emitting element using Comparative Compound 1-b are each defined as 1.0.

**Table 3**

| | Structural formula | Dipole moment (D) | Light emission efficiency | Element life |
|---|---|---|---|---|
| Exemplary Compound A2 | 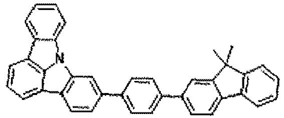 | 0.79 | 1.10 | 1.1 |
| Comparative Compound 1-b | 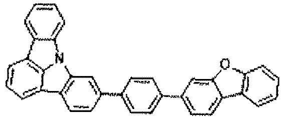 | 0.94 | 1.00 | 1.0 |

In Table 3, the light emission efficiency and the element life of an organic light-emitting element using Exemplary Compound A2 are excellent values compared to an organic light-emitting element using Comparative Compound 1-b. This may be a difference caused by that the structure of Exemplary Compound A2 corresponding to the B is a fluorene skeleton but the structure of Comparative Compound 1-b corresponding to B is a dibenzofuran skeleton. Although the B of Comparative Compound 1-b is a heterocyclic skeleton, the B of Exemplary Compound A2 is a fluorene skeleton, and thereby the polarity of the whole molecule of Exemplary Compound A2 becomes smaller. It is inferred that as a result, energy transfer between Exemplary Compound A2 and the phosphorescent dopant is more efficiently performed, and thereby excellent light emission efficiency and element life are exhibited.

The polarization of a molecule can be estimated from the size of the permanent dipole moment. The organic compound according to the present embodiment may have a permanent dipole moment of 0.9 or less, 0.8 or less, or 0.7 or less.

Furthermore, in the organic compound according to the present embodiment, the substituent included in the general formula (3) or (4) can be a substituent composed of a hydrocarbon. Specifically, the substituent is a substituted or unsubstituted alkyl group or a substituted or unsubstituted aryl group and can be an alkyl group having 1 or more and 4 or less carbon atoms or an aryl group having 6 or more and 12 or less carbon atoms. Specifically, the substituent can be a methyl group, an ethyl group, an iso-propyl group, an iso-butyl group, a tert-butyl group, a cyclohexyl group, or a phenyl group and can be a methyl group or a tert-butyl group.

Furthermore, the organic compound according to the present embodiment can be used in a hole-transporting layer, an electron-blocking layer, a light-emitting layer, or another functional layer of an organic light-emitting element and in particular, can be used as a host material of a light-emitting layer. Among them, the organic compound according to the present embodiment has a high T1 energy and therefore can be used as a host material of a light-emitting layer using the triplet state of a phosphorescent dopant, a delayed fluorescent material, or the like for light emission. This is because a high T1 energy accelerates energy transfer to the guest material and thereby can suppress generation of quencher molecules. As a result, an organic light-emitting element using the organic compound according to the present embodiment exhibits excellent light emission efficiency. In addition, an organic light-emitting element using the organic compound according to the present embodiment exhibits an excellent element life.

The organic compound according to the present embodiment has an indolocarbazole skeleton represented by the general formula (2), thereby has a highest occupied molecular orbital (HOMO) energy (close to the vacuum level) and excellent hole transportability, and therefore can be used as a mixture with a material having electron transportability.

Here, the "excellent electron transportability" may be an excellent material having a higher electron mobility than the hole mobility.

Another embodiment of the organic compound according to the present embodiment will now be described.
(1-3) In the general formula (3), any one of R₂₀₂, R₂₀₃, R₂₀₄, R₂₀₅, R₂₀₆, or R₂₀₇ binds to L, and in the general formula (4), any one of R₃₀₂, R₃₀₃, R₃₀₄, R₃₀₅, R₃₀₆, or R₃₀₇ binds to L.
(1-4) In the general formula (1), L is composed of a metaphenylene group having a binding site at the meta-position.
(1-5) In the general formula (1), L is a direct bond.
(1-6) In the general formula (3), at least one of R₂₀₉ or R₂₁₀ includes a substituent.

The characteristics above will now be described.

(1-3) In the general formula (3), any one of R₂₀₂, R₂₀₃, R₂₀₄, R₂₀₅, R₂₀₆, or R₂₀₇ binds to L, and in the general formula (4), any one of R₃₀₂, R₃₀₃, R₃₀₄, R₃₀₅, R₃₀₆, or R₃₀₇ binds to L.

In the organic compound according to the present embodiment, in the general formula (3), any one of R₂₀₂, R₂₀₃, R₂₀₄, R₂₀₅, R₂₀₆, or R₂₀₇ can bind to L, and in the general formula (4), any one of R₃₀₂, R₃₀₃, R₃₀₄, R₃₀₅, R₃₀₆, or R₃₀₇ can bind to L, from the viewpoint of synthesis.

In particular, in the general formula (3), R₂₀₃ or R₂₀₆ can bind to L, and in the general formula (4), R₃₀₃ or R₃₀₆ can bind to L, from the viewpoint of the T1 energy.

The fluorene skeleton can more suppress the spread of conjugation by binding to L at the position 3 or the position 6 (corresponding to R₂₀₃ and R₂₀₆, respectively) and therefore can exhibit a higher T1 energy. Table 4 shows comparison between the T1 wavelengths of Exemplary Compound B5 and Exemplary Compound A6.

**Table 4**

| | Molecular structure | T1 (nm) |
|---|---|---|
| Exemplary Compound B5 | 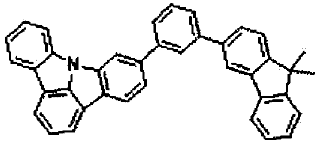 | 439 |
| Exemplary Compound A6 | 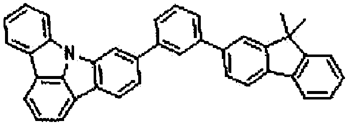 | 445 |

Table 4 demonstrates that the T1 wavelength of Exemplary Compound B5 binding to the linking group at the position 3 of the fluorene skeleton is shorter than the T1 wavelength of Exemplary Compound A6 binding to the linking group at the position 2 of the fluorene skeleton. Accordingly, in the organic compound according to the present embodiment, in the general formula (3), R₂₀₃ or R₂₀₆ can bind to L, and in the general formula (4), R₃₀₃ or R₃₀₆ can bind to L.

(1-4) In the general formula (1), L is composed of a metaphenylene group having a binding site at the meta-position.

The organic compound according to the present embodiment exhibits a higher T1 energy by that in the general formula (1), L is composed of a metaphenylene group having a binding site at the meta-position. Table 5 shows comparison between the T1 wavelengths of Exemplary Compound A6 and Exemplary Compound A2.

**Table 5**

| | Molecular structure | T1 (nm) |
|---|---|---|
| Exemplary Compound A6 | 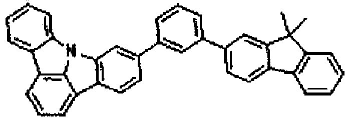 | 445 |
| Exemplary Compound A2 | 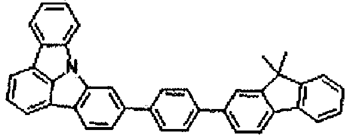 | 465 |

Table 5 demonstrates that the T1 wavelength of Exemplary Compound A6 in which L is composed of a metaphenylene group having a binding site at the meta-position is shorter than the T1 wavelength of Exemplary Compound A2 in which L does not include a metaphenylene group having a binding site at the meta-position. Accordingly, in the organic compound according to the present embodiment, in the general formula (1), L can be composed of a metaphenylene group having a binding site at the meta-position. In particular, the general formula (1) can be the general formula (6) or the general formula (8) or can be the general formula (8).

(1-5) In the general formula (1), L is a direct bond.

In the organic compound according to the present embodiment, in the general formula (1), when L is a direct bond, the interaction between the organic compound molecules according to the present embodiment can be reduced.

In the organic compound according to the present embodiment, the indolocarbazole skeleton represented by the general formula (1) is highly planar, and the skeleton molecules easily interact with each other. In contrast, the fluorene skeleton represented by the general formula (3) and the spirofluorene skeleton represented by the general formula (4) are less planar (bulky) skeletons compared to the indolocarbazole skeleton. In the general formula (1), when L is a direct bond, the organic compound has a structure in which a less planar (bulky) fluorene skeleton or spirofluorene skeleton comes near the highly planar indolocarbazole skeleton. As a result, the intermolecular interaction of the indolocarbazole skeleton can be reduced.

Because of the above reasons, when L is a direct bond, the organic compound according to the present embodiment hardly aggregates, and thereby the compatibility between the organic compound according to the present embodiment and the phosphorescent dopant is improved. As a result, the energy transfer between the organic compound according to the present embodiment and the phosphorescent dopant is accelerated, and thereby an organic light-emitting element using the organic compound according to the present embodiment exhibits more excellent light emission efficiency. In addition, an organic light-emitting element using the organic compound according to the present embodiment exhibits a more excellent element life.

In particular, in the organic compound according to the present embodiment, the general formula (1) can be the general formula (5) or the general formula (7) and may be the general formula (7).

(1-6) In the general formula (3), at least one of R₂₀₉ or R₂₁₀ includes a substituent.

The organic compound according to the present embodiment becomes a bulkier skeleton because by that in the general formula (3), at least one of R₂₀₉ or R₂₁₀ has a substituent, and thereby the interaction between the organic compound molecules according to the present embodiment can be more reduced. This is because that in the general formula (3), when R₂₀₉ or R₂₁₀ includes a substituent, the substituent is arranged in a direction perpendicular to a conjugate plane.

Table 6 shows comparison among Exemplary Compounds A5 to A7, which are organic compounds according to the present embodiment. Light emission efficiency and Element life in Table 6 are respectively values when the light emission efficiency and the element life of an organic light-emitting element using Exemplary Compound A7 are each defined as 1.0.

In Table 6, the size in the z direction is the size in the z direction (height direction) when the conjugate plane of the fluorene skeleton is defined as the xy-plane. The larger this value, the bulkier the skeleton.

**Table 6**

| | Molecular structure | Size in z direction (pm) | Light emission efficiency | Element life |
|---|---|---|---|---|
| Exemplary Compound A5 | 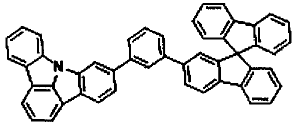 | 9.05 | 1.10 | 1.2 |
| Exemplary Compound A6 | 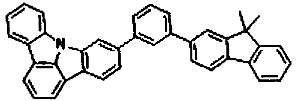 | 4.35 | 1.05 | 1.1 |
| Exemplary Compound A7 | 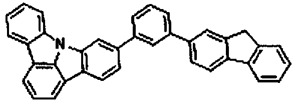 | 1.76 | 1.00 | 1.0 |

Table 6 demonstrates that in the general formula (3), the light emission efficiency and the element life are improved by that at least one of R₂₀₉ or R₂₁₀ has a bulkier substituent than a hydrogen atom. This is inferred that the interaction between molecules is reduced by the bulky substituent of the molecule.

Based on the above results, in the general formula (3), at least one of R₂₀₉ or R₂₁₀ can be a bulkier substituent than a hydrogen atom, such as a deuterium atom, an alkyl group having 1 or more and 4 or less carbon atoms, and an aryl group having 6 or more and 12 or less carbon atoms. In addition, in the general formula (1), B can be a spirofluorene skeleton represented by the general formula (4).

The T1 wavelength, the permanent dipole moment, and the size in the z direction in Tables 1 to 6 were calculated using molecular orbital calculation.

As the molecular orbital calculation method, density functional theory (DFT), which is widely used presently, is used. The generic function used is B3LYP, and the basis function used is 6-31G*. The molecular orbital calculation method is carried out by Gaussian09 (Gaussian09, Revision C.01, M.J. Frisch, G.W. Trucks, H.B. Schlegel, G.E. Scuseria, M.A. Robb, J.R. Cheeseman, G. Scalmani, V. Barone, B. Mennucci, G.A. Petersson, H. Nakatsuji, M. Caricato, X. Li, H.P. Hratchian, A.F. Izmaylov, J. Bloino, G. Zheng, J.L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J.A. Montgomery, Jr., J.E. Peralta, F. Ogliaro, M. Bearpark, J.J. Heyd, E. Brothers, K.N. Kudin, V.N. Staroverov, T. Keith, R. Kobayashi, J. Normand, K. Raghavachari, A. Rendell, J.C. Burant, S.S. Iyengar, J. Tomasi, M. Cossi, N. Rega, J.M. Millam, M. Klene, J.E. Knox, J.B. Cross, V. Bakken, C. Adamo, J. Jaramillo, R. Gomperts, R.E. Stratmann, O. Yazyev, A.J. Austin, R. Cammi, C. Pomelli, J.W. Ochterski, R.L. Martin, K. Morokuma, V.G. Zakrzewski, G.A. Voth, P. Salvador, J.J. Dannenberg, S. Dapprich, A.D. Daniels, O. Farkas, J.B. Foresman, J.V. Ortiz, J. Cioslowski, and D.J. Fox, Gaussian, Inc., Wallingford CT, 2010), which is widely used presently. Hereinafter, the molecular orbital calculations in the present specification use the same technique.

In the present specification, the highest occupied molecular orbital (HOMO) energy and the lowest unoccupied molecular orbital (LUMO) energy can be calculated using ionization potential and bandgap.

The HOMO energy can be estimated by measuring the ionization potential. The ionization potential can be measured with a measurement apparatus such as AC-3, after dissolving the compound as a measurement target in a solvent such as toluene or vapor-depositing the compound as a measurement target on a substrate such as glass.

The LUMO energy can be calculated using the values of bandgap and ionization potential. The LUMO energy can be estimated by subtracting the ionization potential value from the bandgap. The bandgap can be measured by dissolving the compound as a measurement target in a solvent such as toluene, irradiating the solution with excitation light, and measuring the absorption edge of the absorption spectrum of the excitation light. The bandgap can be measured by vapor-depositing the compound as a measurement target on a substrate such as glass, irradiating the deposited film with excitation light, and measuring the absorption edge of the absorption spectrum of the excitation light by the deposited film.

The LUMO energy can also be estimated from the reduction potential. For example, one-electron reduction potential is estimated by cyclic voltammetry (CV) measurement. The measurement by CV is carried out, for example, in a 0.1 M tetrabutylammonium hyperchloric acid DMF solution using Ag/Ag+ as the reference electrode, Pt as the counter electrode, and glassy carbon as the working electrode. LUMO can be estimated by adding the difference between the obtained reduction potential of the compound and the reduction potential of ferrocene to -4.8 eV.

Specific examples of the organic compound according to the present embodiment are shown below, but the present embodiment is not limited thereto.

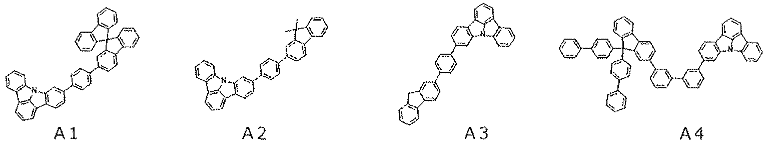

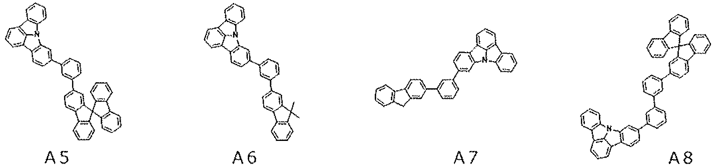

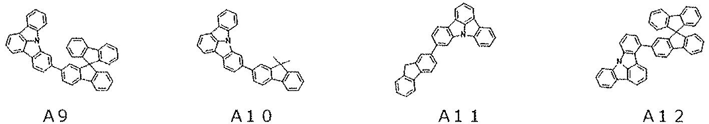

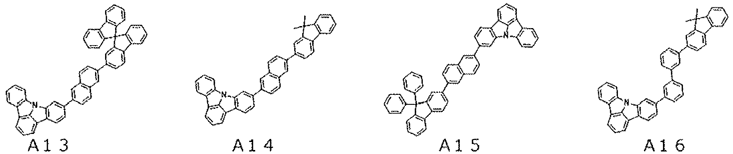

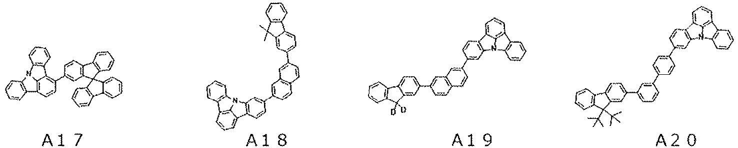

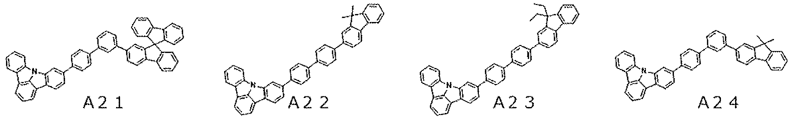

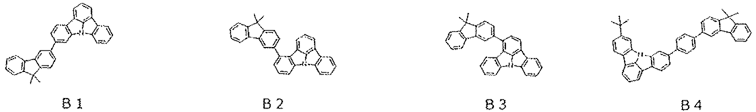

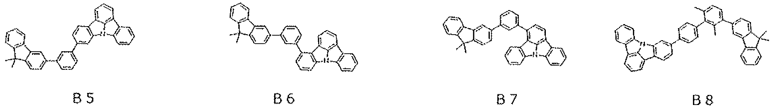

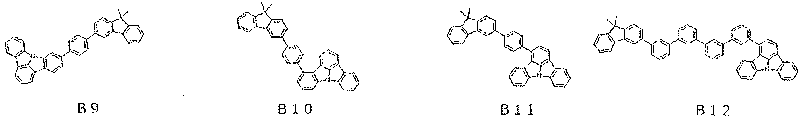

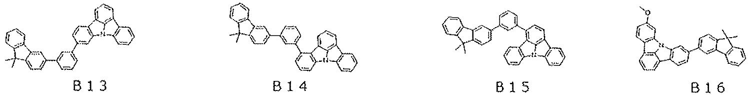

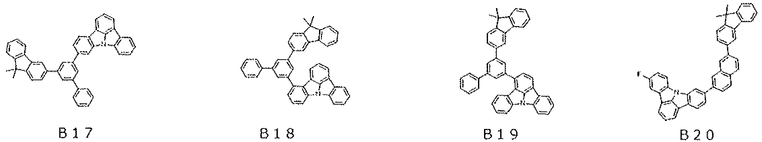

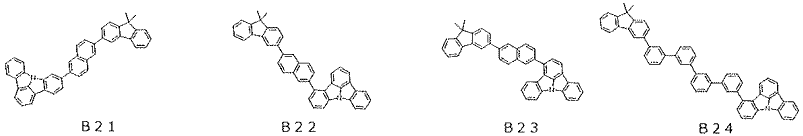

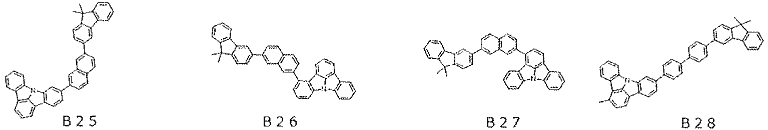

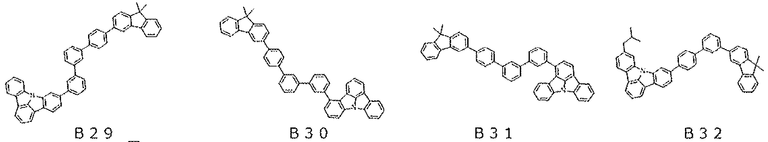

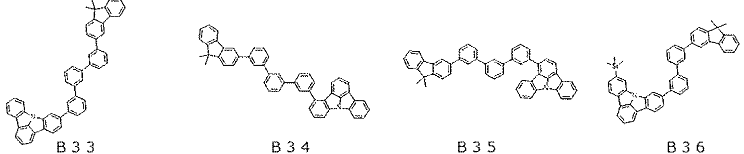

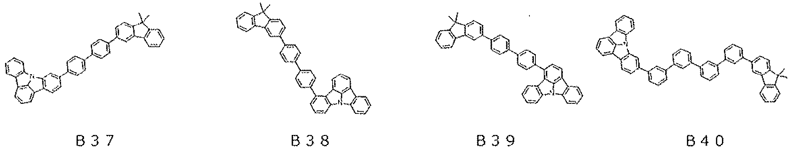

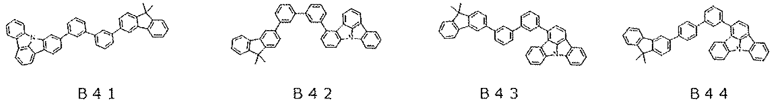

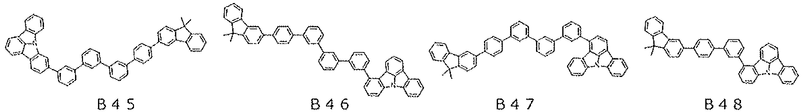

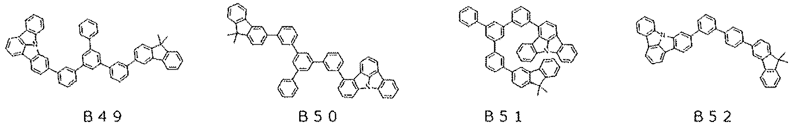

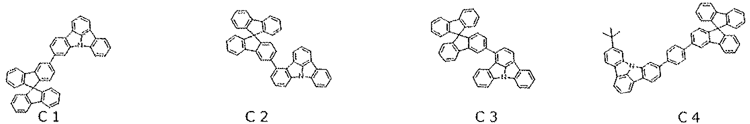

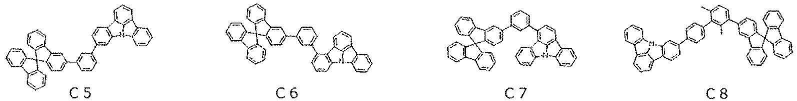

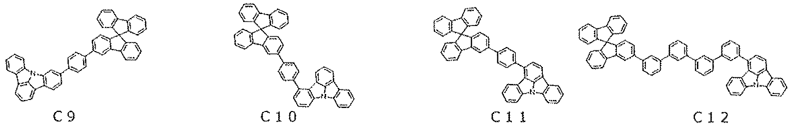

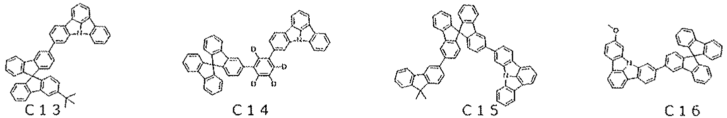

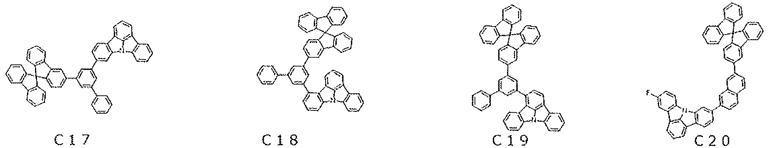

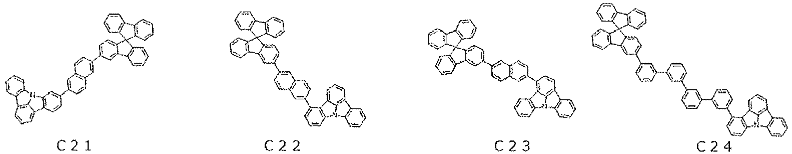

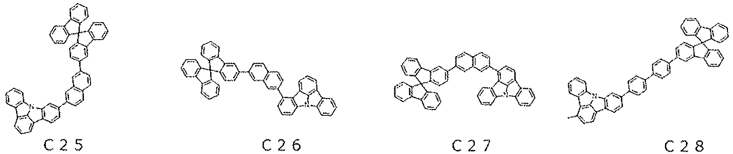

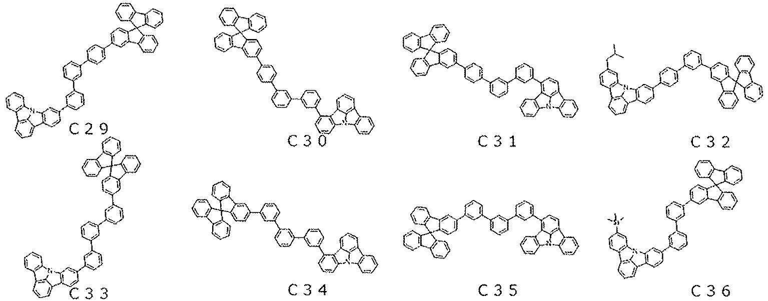

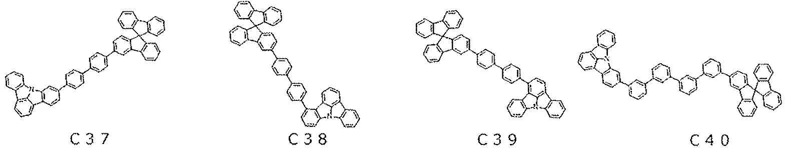

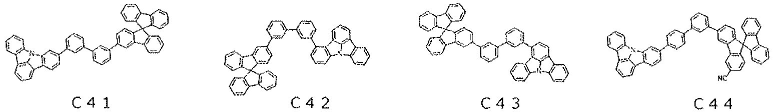

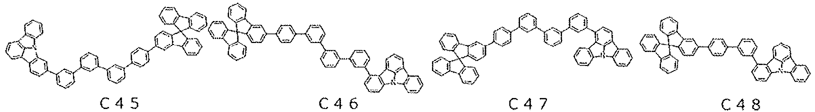

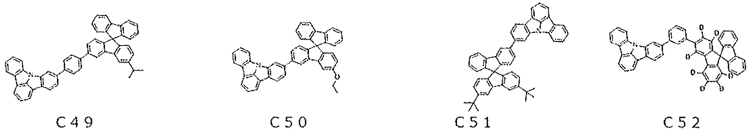

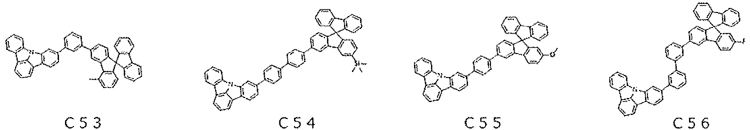

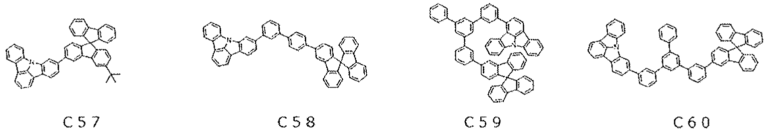

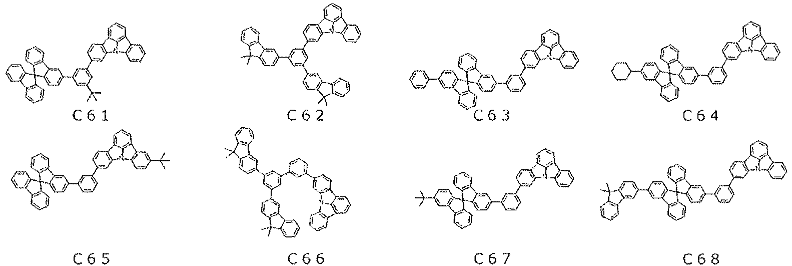

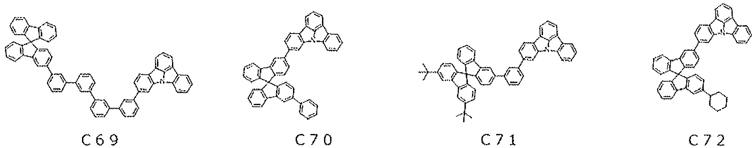

Exemplary Compounds belonging to the group A are compounds in the general formula (3) in which L binds at R₂₀₂ or R₂₀₇. In the compounds of the group A, the molecular orbital tends to spread over the whole of the molecule to exert an effect of providing excellent charge transportability.

Exemplary Compounds belonging to the group B are compounds in the general formula (3) in which L binds at R₂₀₃ or R₂₀₆, and R₂₀₉ and R₂₁₀ are methyl groups. The compounds of the group B have high T1 energies and relatively small molecular weights to exert an effect of providing excellent sublimability.

Exemplary Compounds belonging to the group C are compounds in the general formula (4) in which L binds at R₃₀₃ or R₃₀₆. The compounds of the group C have high T1 energies and exerts an effect of improving the compatibility between the organic compound according to the present embodiment and the light-emitting dopant.

### [2] Organic light-emitting element

The organic light-emitting element of the present embodiment will now be described. The organic light-emitting element of the present embodiment at least includes a first electrode, a second electrode, an organic compound layer disposed between these electrodes.

One of the first electrode and the second electrode is a positive electrode, and the other is a negative electrode. In the organic light-emitting element of the present embodiment, the organic compound layer may be a single layer or a laminate composed of a plurality of layers as long as it includes a light-emitting layer. Here, when the organic compound layer is a laminate composed of a plurality of layers, the organic compound layer may include, in addition to a light-emitting layer, a hole-injection layer, a hole-transporting layer, an electron-blocking layer, a hole/exciton-blocking layer, an electron-transporting layer, an electron-injection layer, or the like. The light-emitting layer may be a single layer or a laminate composed of a plurality of layers.

In the organic light-emitting element of the present embodiment, at least one organic compound layer contains the organic compound according to the present embodiment. Specifically, the organic compound according to the present embodiment is contained in any one of the above-mentioned light-emitting layer, hole-injection layer, hole-transporting layer, electron-blocking layer, hole/exciton-blocking layer, electron-transporting layer, electron-injection layer, and so on. The organic compound according to the present embodiment can be contained in the hole-transporting layer, the electron-blocking layer, the hole/exciton-blocking layer, the electron-transporting layer, or the light-emitting layer. The organic compound according to the present embodiment may be contained in the light-emitting layer.

In the organic light-emitting element of the present embodiment, the organic compound according to the present embodiment is contained in a light-emitting layer, the light-emitting layer may be a layer composed of the organic compound according to the present embodiment only or may be a layer composed of the organic compound according to the present embodiment and another compound. Here, when the light-emitting layer is a layer composed of the organic compound according to the present embodiment and another compound, the organic compound according to the present embodiment may be used as the host material of the light-emitting layer or may be used as the guest material. Alternatively, the organic compound according to the present embodiment may be used as the assist material that can be contained in a light-emitting layer. The organic compound of the present embodiment can be used as the host material of a light-emitting layer of a system using a triplet excited state such as phosphorescence or delayed fluorescence for light emission. Accordingly, the light-emitting layer can further include a phosphorescent dopant. The organic compound of the present embodiment can be used as a mixture with an electron-transporting host material. Accordingly, the light-emitting layer can further contain an electron-transporting compound.

Here, the host material is the compound with the largest mass ratio among the compounds constituting the light-emitting layer. In another viewpoint, the host material is said as a material having a higher T1 energy than the T1 energy of the guest material. The guest material is a compound with a smaller mass ratio than that of the host material among the compounds constituting the light-emitting layer and is a compound mainly bearing light emission. In another viewpoint, the guest material is a compound having a lower T1 energy than that of the host material. The assist material is a compound of which the mass ratio is smaller than that of the host among the compounds constituting the light-emitting layer and assists the light emission of the guest. In another viewpoint, the assist material is a compound having a lower T1 energy than that of the host material. The assist material may have a higher T1 energy than that of the guest material. The assist material is also called a second host. The host material can also be called a first compound, and the assist material can also be called a second compound.

The concentration of the host material in the light-emitting layer according to the present embodiment is preferably 10 mass% or more and 90 mass% or less based on the total mass of the light-emitting layer, more preferably 20 mass% or more and 80 mass% or less, and further preferably 30 mass% or more and 70 mass% or less. The concentration of the guest material relative to the host material is 0.01 mass% or more and 50 mass% or less or may be 0.1 mass% or more or 20 mass% or less, based on the total mass of the constituent materials of the light-emitting layer. From the viewpoint of suppressing concentration quenching, the concentration of the guest material can be 10 mass% or less.

The guest material may be contained uniformly throughout the layer in which the host material is the matrix or may be contained with a concentration gradient. The guest material may be contained partially in a specific region of the light-emitting layer so that the layer has a region of the host material only without containing the guest.

The light-emitting layer of the present embodiment may be a single layer or multiple layers and can mix colors by including light-emitting materials having different emission colors. The multilayer means a state in which a light-emitting layer and another light-emitting layer are laminated. In this case, the emission color of the organic light-emitting element is not specifically limited and, more specifically, may be white or may be a neutral color. In the case of white, for example, if the emission color of the light-emitting layer is blue, the other light-emitting layer emits light of a color different from blue, i.e., emits a green or red color. The film-forming method is deposition or coating film formation.

Examples of the element configuration of the organic light-emitting element of the present embodiment include a multilayer configuration in which electrode layers and an organic compound layer are stacked sequentially on a substrate as shown in the following (1) to (6). In every element configuration, the organic compound layer includes invariably a light-emitting layer containing a light-emitting material:
(1) positive electrode/light-emitting layer/negative electrode;
(2) positive electrode/hole-transporting layer/light-emitting layer/electron-transporting layer/negative electrode;
(3) positive electrode/hole-transporting layer/light-emitting layer/electron-transporting layer/electron-injection layer/negative electrode;
(4) positive electrode/hole-injection layer/hole-transporting layer/light-emitting layer/electron-transporting layer/negative electrode;
(5) positive electrode/hole-injection layer/hole-transporting layer/light-emitting layer/electron-transporting layer/electron-injection layer/negative electrode; and
(6) positive electrode/hole-transporting layer/electron-blocking layer/light-emitting layer/hole-blocking layer/electron-transporting layer/negative electrode.

However, these examples of the element configuration are merely very basic element configurations, and the element configuration is not limited thereto. For example, it is possible to adopt various layer configurations such as a configuration in which an insulating layer, an adhesive layer, or an interference layer is arranged at the interface between an electrode and an organic compound layer; a configuration in which an electron-transporting layer or a hole-transporting layer is constituted of two layers having different ionization potentials; and a configuration in which a light-emitting layer is constituted of two layers of different light-emitting materials.

In the element configurations shown in the above (1) to (6), the configuration of (6) having both an electron-blocking layer and a hole-blocking layer may be used. That is, in the configuration (6) having both the electron-blocking layer and the hole-blocking layer, since both hole and electron carriers can be reliably confined in the light-emitting layer, the organic light-emitting element can have high light emission efficiency without causing carrier leakage.

The extraction mode (element configuration) of light output from the light-emitting layer may be a so-called bottom emission system that extracts light from the electrode on the substrate side or may be a so-called top emission system that extracts light from the opposite side to the substrate. Alternatively, a double-sided extraction system that extracts light from both the substrate side and the opposite side to the substrate can also be adopted.

The organic compound according to the present embodiment can be used as a constituent material of an organic compound layer other than the light-emitting layer constituting the organic light-emitting element of the present embodiment. Specifically, the organic compound according to the present embodiment may be used as a constituent material for an electron-transporting layer, an electron-injection layer, a hole-transporting layer, a hole-injection layer, a hole-blocking layer, or the like. In this case, the emission color of the organic light-emitting element is not specifically limited and, more specifically, may be white or may be a neutral color.

### Other compound

The organic light-emitting element according to the present embodiment can be used together with a known low-molecular or high-molecular hole-injecting or hole-transporting compound, a compound functioning as a host material, a light-emitting compound, an electron-injecting compound, an electron-transporting compound, or the like, as needed. Examples of these compounds include the following.

The hole-injecting/transporting material can be a material having a high hole mobility so that holes can be easily injected from the positive electrode and that the injected holes can be transported to the light-emitting layer. In addition, in order to suppress the degradation of film quality, such as crystallization, in the organic light-emitting element, a material having a high glass transition temperature can be used. Examples of the low-molecular and high-molecular materials having hole-injecting/transporting ability include a triarylamine derivative, an arylcarbazole derivative, a phenylenediamine derivative, a stilbene derivative, a phthalocyanine derivative, a porphyrin derivative, poly(vinylcarbazole), poly(thiophene), and other conductive polymers. The hole-injecting/transporting material can also be used in an electron-blocking layer. Specific examples of the compound that is used as the hole-injecting/transporting material are shown below, but the compound is not limited thereto.

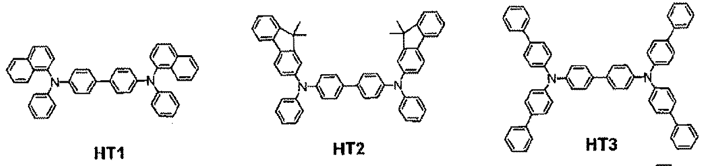

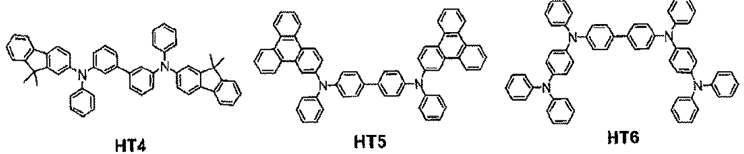

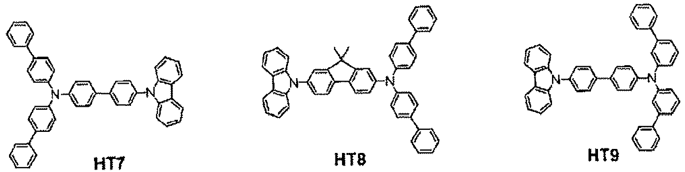

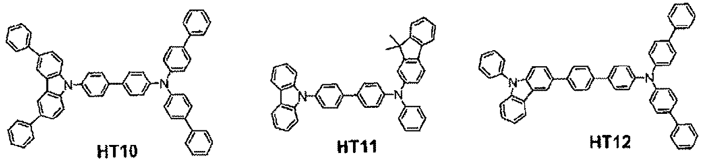

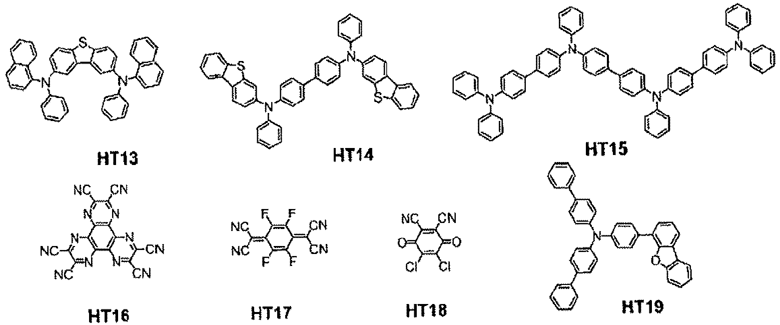

Examples of the light-emitting material that is mainly involved in the light-emitting function include a condensed ring compound (e.g., a fluorene derivative, a naphthalene derivative, a pyrene derivative, a perylene derivative, a tetracene derivative, an anthracene derivative, and rubrene), a quinacridone derivative, a coumarin derivative, a stilbene derivative, an organic aluminum complex such as tris(8-quinolinolato)aluminum, an iridium complex, a platinum complex, a rhenium complex, a copper complex, a europium complex, a ruthenium complex, and a polymer derivative such as poly(phenylenevinylene) derivative, a poly(fluorene) derivative, and a poly(phenylene) derivative. Specific examples of the compound that is used as the light-emitting material are shown below, but the compound is not limited thereto.

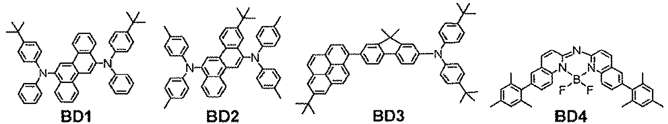

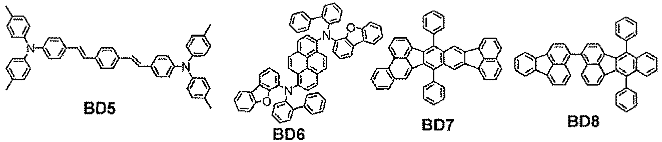

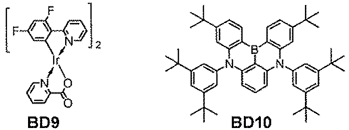

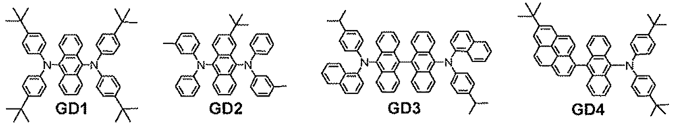

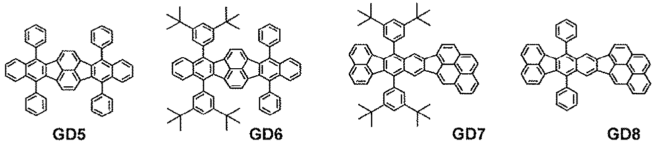

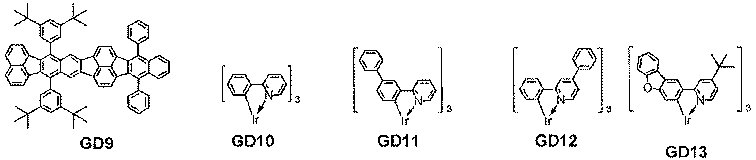

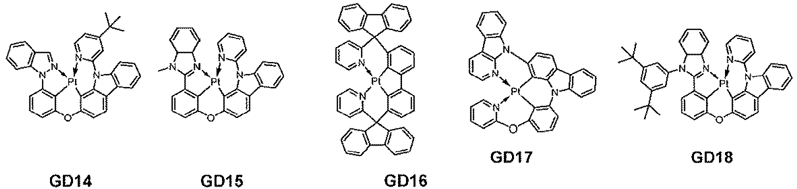

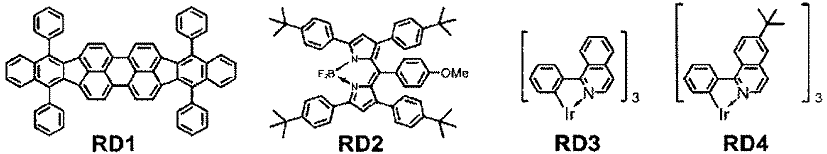

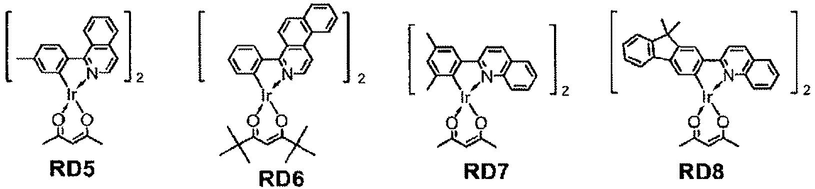

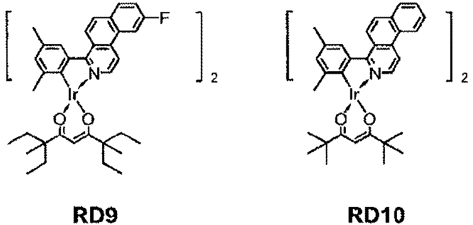

The light-emitting layer may contain a third component that is a compound other than the organic compound according to the present embodiment as a host material or assist material. Examples of the third component include a carbazole derivative, an azine derivative, a xanthone derivative, a dibenzofuran derivative, a dibenzothiophene derivative, an organic aluminum complex such as tris(8-quinolinolato)aluminum, and an organic beryllium complex, in addition to an aromatic hydrocarbon compound and a derivative thereof.

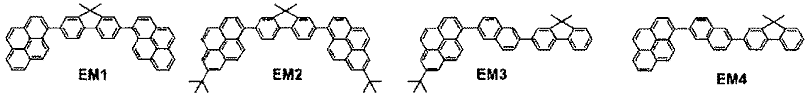

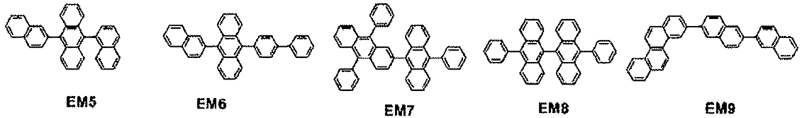

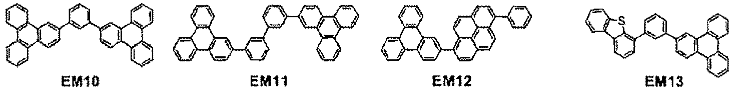

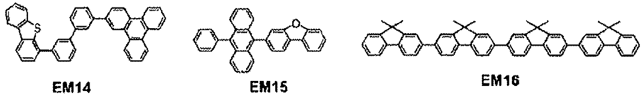

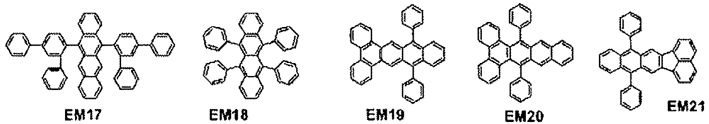

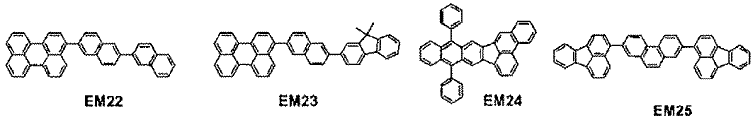

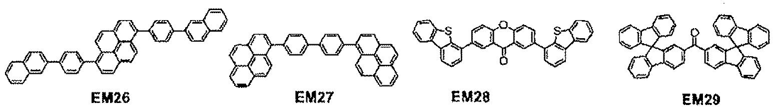

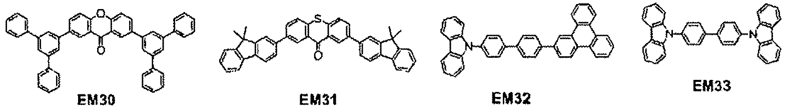

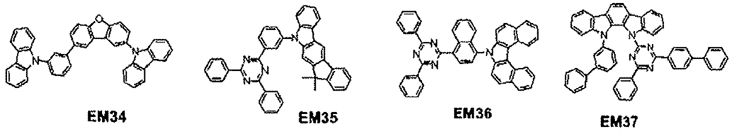

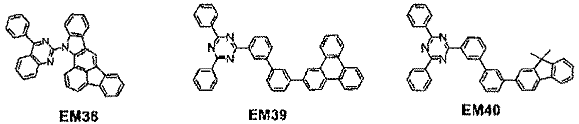

The electron-transporting material can be arbitrarily selected from materials that can transport electrons injected from the negative electrode to the light-emitting layer and is selected considering, for example, the balance with the hole mobility of the hole-transporting material. Examples of the material having electron transporting ability include an oxadiazole derivative, an oxazole derivative, a pyrazine derivative, a triazole derivative, a triazine derivative, a quinoline derivative, a quinoxaline derivative, a phenanthroline derivative, an organic aluminum complex, a condensed ring compound (e.g., a fluorene derivative, a naphthalene derivative, a chrysene derivative, and an anthracene derivative). Furthermore, the above-mentioned electron-transporting material is also used in the hole-blocking layer. Specific examples of the compound that is used as the electron-transporting material are shown below, but the compound is not limited thereto.

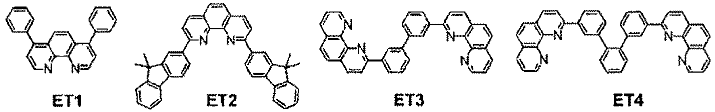

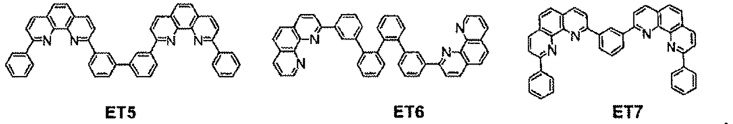

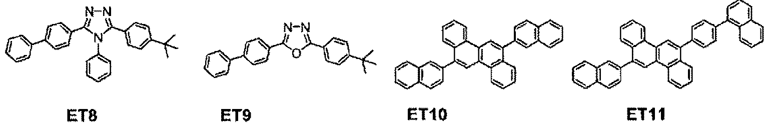

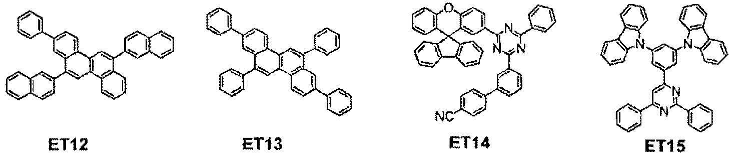

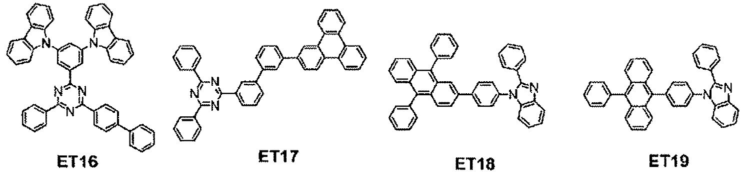

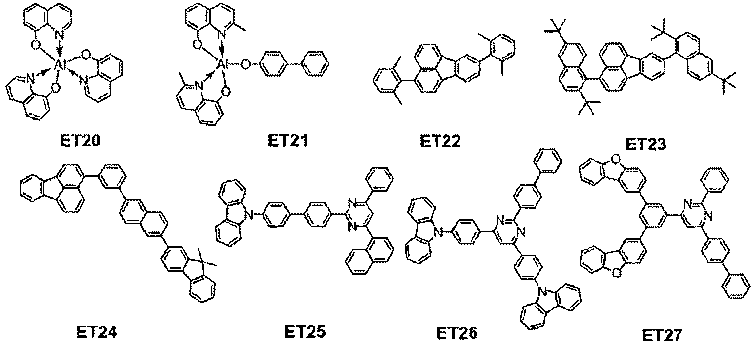

The electron-injecting material can be arbitrarily selected from materials that allows easy electron injection from the negative electrode and is selected considering, for example, the balance with the hole injection property. An n-type dopant and a reducible dopant are also included in the organic compound, and examples thereof include a compound including an alkali metal, such as lithium fluoride, a lithium complex such as lithium quinolinol, a benzimidazolidene derivative, an imidazolidene derivative, a fulvalene derivative, and an acridine derivative. The electron-injecting material can also be used together with the above-mentioned electron-transporting material.

### [3] Configuration of organic light-emitting element

The organic light-emitting element is provided by forming an insulation layer, a first electrode, an organic compound layer, and a second electrode on a substrate. On the second electrode, a protection layer, a color filter, a microlens, and so on may be provided. When a color filter is provided, a planarization layer may be arranged between the color filter and a protection layer. The planarization layer can be constituted of an acrylic resin or the like, which is the same as when a planarization layer is provided between a color filter and a microlens. One of the first electrode and the second electrode may be a positive electrode, and the other may be a negative electrode.

### Substrate

Examples of the substrate include quartz, glass, a silicon wafer, a resin, and a metal. A switching element such as a transistor and wiring may be provided on the substrate, and an insulation layer may be provided thereon. The material for the insulation layer is not limited as long as a contact hole can be formed such that wiring can be formed between the insulation layer and the first electrode and that insulation from unconnected wiring can be ensured. For example, a resin such as polyimide, silicon oxide, and silicon nitride can be used.

### Electrode

A pair of electrodes can be used. The pair of electrodes may be a positive electrode and a negative electrode.

When an electric field is applied in the direction in which the organic light-emitting element emits light, the electrode with a higher potential is the positive electrode, and the other is the negative electrode. Alternatively, it can be said that the electrode that supplies holes to the light-emitting layer is the positive electrode and the electrode that supplies electrons is the negative electrode.

The constituent material of the positive electrode can have as large a work function as possible. For example, a metal simple substance such as gold, platinum, silver, copper, nickel, palladium, cobalt, selenium, vanadium, and tungsten, a mixture containing such a metal simple substance, an alloy of a combination of these metal simple substances, or a metal oxide such as tin oxide, zinc oxide, indium oxide, indium tin oxide (ITO), and indium zinc oxide can be used. In addition, a conductive polymer such as polyaniline, polypyrrole, or polythiophene can also be used.

These electrode substances may be used alone or in combination of two or more. The positive electrode may be constituted of one layer or may be constituted of a plurality of layers.

When the electrode is used as a reflective electrode, for example, chromium, aluminum, silver, titanium, tungsten, molybdenum, or an alloy or laminate thereof can be used. The above-mentioned materials can also function as a reflective film not having a role as an electrode. When the electrode is used as a transparent electrode, a transparent conductive layer of an oxide such as indium tin oxide (ITO) and indium zinc oxide can be used, but the material is not limited thereto.

The electrode can be formed by photolithography.

In contrast, the constituent material of the negative electrode can be a material having a small work function, and examples thereof include an alkali metal such as lithium, an alkaline earth metal such as calcium, a metal simple substance such as aluminum, titanium, manganese, silver, lead, and chromium, and a mixture including such a material. Alternatively, an alloy of a combination of these materials can also be used. For example, magnesium-silver, aluminum-lithium, aluminum-magnesium, silver-copper, or zinc-silver can be used. A metal oxide such as indium tin oxide (ITO) can also be used. These electrode materials may be used alone or in combination of two or more. The negative electrode may have a single layer configuration or may have a multi-layer configuration. In particular, silver can be used, and a silver alloy may be used for reducing aggregation of silver. The alloy ratio is not limited as long as the aggregation of silver can be reduced. For example, silver : another material may be 1 : 1, 3 : 1, or the like.

The negative electrode may be used in a top emission element using a conductive layer of an oxide such as ITO or may be used in a bottom emission element using a reflective electrode of aluminum (Al) or the like, and the use thereof is not specifically limited. The formation method of the negative electrode is not specifically limited, but may be a DC or AC sputtering method, which provides high film coverage and easily provides lower resistance.

### Organic compound layer

The organic compound layer may be formed as a single layer or multiple layers. The organic compound layer composed of a plurality of layers may be referred to as a hole-injection layer, a hole-transporting layer, an electron-blocking layer, a light-emitting layer, a hole-blocking layer, an electron-transporting layer, or an electron-injection layer based on its function. The organic compound layer is mainly composed of an organic compound and may contain an inorganic atom or an inorganic compound, such as copper, lithium, magnesium, aluminum, iridium, platinum, molybdenum, and zinc. The organic compound layer may be disposed between the first electrode and the second electrode and may be arranged in contact with the first electrode and the second electrode.

The organic compound layer (such as a hole-injection layer, a hole-transporting layer, an electron-blocking layer, a light-emitting layer, a hole-blocking layer, an electron-transporting layer, and an electron-injection layer) constituting an organic light-emitting element according to an embodiment of the present disclosure is formed by the method shown below.

In the organic compound layer constituting an organic light-emitting element according to an embodiment of the present disclosure, a dry process such as vacuum deposition, ionization deposition, sputtering, and plasma processing can be used. Instead of the dry process, a wet process that forms a layer by a known coating method using an appropriate solvent for dissolution (e.g., spin coating, casting, microgravure coating, gravure coating, bar coating, roll coating, wire bar coating, dip coating, spray coating, screen printing, flexographic printing, offset printing, inkjet printing, capillary coating, and nozzle coating) can also be used. In particular, vacuum deposition, ionization deposition, inkjet printing, and nozzle coating can be used for manufacturing organic light-emitting elements with large areas.

Here, the layer formed by vacuum deposition, solution coating, or the like hardly causes crystallization and has excellent temporal stability. When a film is formed by coating, the film can also be formed in combination with an appropriate binder resin.

Examples of the binder resin include, but not limited to, a polyvinylcarbazole resin, a polycarbonate resin, a polyester resin, an ABS resin, an acrylic resin, a polyimide resin, a phenolic resin, an epoxy resin, a silicon resin, and a urea resin.

These binder resins may be used alone as a homopolymer or copolymer or may be used as a mixture of two or more. Furthermore, as needed, a known additive, such as a plasticizer, an oxidation inhibitor, and an ultraviolet absorber, may be used in combination.

The thickness of each layer in the organic light-emitting element can usually be 1 nm or more and 10 µm or less. In particular, the thickness of the light-emitting layer of the organic compound layer can be 10 nm or more and 100 nm or less for obtaining effective light-emitting characteristics.

### Protection layer

A protection layer may be disposed on the second electrode. For example, infiltration of water and the like into the organic compound layer can be reduced by adhering glass provided with a moisture absorbent onto the second electrode, and thereby the occurrence of display defects can be reduced. In another embodiment, the infiltration of water and the like into the organic compound layer may be reduced by providing a passivation film of silicon nitride or the like on the second electrode. For example, a second electrode is formed and is then transported to another chamber without breaking the vacuum, and a silicon nitride film of a thickness of 2 µm may be formed by a CVD method as a protection layer. After the film formation by a CVD method, a protection layer may be provided by an atomic layer deposition method (ALD method). The material for the film by the ALD method is not limited and may be silicon nitride, silicon oxide, aluminum oxide, or the like. A silicon nitride may be further formed by a CVD method on the film formed by the ALD method. The film formed by the ALD method may have a smaller thickness than the film formed by the CVD method, specifically, 50% or less or 10% or less of the thickness of the latter.

### Color filter

A color filter may be provided on the protection layer. For example, a color filter considered the size of the organic light-emitting element may be disposed on another substrate and be attached to the substrate provided with the organic light-emitting element, or a color filter may be patterned on the above-described protection layer by photolithography. The color filter may be constituted of a polymer.

### Planarization layer

A planarization layer may be present between the color filter and the protection layer. The planarization layer is provided for the purpose of reducing the unevenness of the lower layer. Without limiting the purpose, it may also be referred to as a material resin layer. The planarization layer may be constituted of an organic compound, and the compound may be a low-molecular-weight or high-molecular-weight compound and can be a high-molecular-weight compound.

Planarization layers may be provided above and below the color filter, and the constituent materials thereof may be the same or different. Specifically, examples of the constituent material include a polyvinylcarbazole resin, a polycarbonate resin, a polyester resin, an ABS resin, an acrylic resin, a polyimide resin, a phenolic resin, an epoxy resin, a silicon resin, and a urea resin.

### Microlens

The organic light-emitting element may include an optical member, such as a microlens, on the light emission side thereof. The microlens can be constituted of an acrylic resin, an epoxy resin, or the like. The microlens may be for the purpose of increasing the amount of light that is extracted from the organic light-emitting element and of controlling the direction of the light to be extracted. The microlens may have a hemispherical shape. When the microlens has a hemispherical shape, among the tangents being in contact with the hemisphere, there is a tangent parallel to the insulation layer, and the contact point between the tangent and the hemisphere is the vertex of the microlens. The vertex of the microlens can be determined in a similar manner for any cross-sectional view. That is, among tangents in contact with the hemisphere of the microlens in the cross-sectional view, there is a tangent that is parallel to the insulation layer, and the contact point between the tangent and the hemisphere is the vertex of the microlens.

A middle point of the microlens can also be defined. In a cross-section of the microlens, a line segment from a point where the shape of an arc ends to a point where the shape of another arc ends is imagined, and the middle point of the line segment can be referred to as a middle point of the microlens. The cross-section for identifying the vertex and the middle point may be a cross-section perpendicular to the insulation layer.

### Opposite substrate

On the planarization layer, an opposite substrate may be provided. The opposite substrate is disposed at a position corresponding to the above-described substrate and is therefore called an opposite substrate. The constituent material of the opposite substrate may be the same as the above-described substrate. When the above-described substrate is a first substrate, the opposite substrate may be a second substrate.

### Pixel circuit

An organic light-emitting apparatus including the organic light-emitting element may include a pixel circuit that is connected to the organic light-emitting element. The pixel circuit may be an active matrix type that independently controls each of a first light-emitting element and a second light-emitting element. The active matrix type circuit may be voltage programming or current programming. The driving circuit includes a pixel circuit for each pixel. The pixel circuit may include a light-emitting element, a transistor that controls the emission luminance of the light-emitting element, a transistor that controls light emission timing, a capacitor that retains the gate voltage of the transistor that controls the emission luminance, and a transistor for connecting to GND without through the light-emitting element.

The light-emitting apparatus includes a display region and a peripheral region arranged in the circumference of the display region. The apparatus includes a pixel circuit in the display region and a display control circuit in the peripheral region. The mobility of the transistor constituting the pixel circuit may be smaller than the mobility of the transistor constituting the display control circuit.

The gradient of the current-voltage characteristics of the transistor constituting the pixel circuit may be smaller than the gradient of the current-voltage characteristics of the transistor constituting the display control circuit. The gradient of current-voltage characteristics can be measured by the so-called Vg-Ig characteristics. The transistor constituting the pixel circuit is a transistor that is connected to a light-emitting element such as the first light-emitting element.

### Pixel

The organic light-emitting apparatus including the organic light-emitting element may include a plurality of pixels. The pixel has sub-pixels that emit different colors. For example, the sub-pixels may have R, G, and B emission colors, respectively.

The pixel emits light in a region also called a pixel aperture. This region is the same as the first region. The pixel aperture may be 15 µm or less and 5 µm or more. More specifically, the pixel aperture may be, for example, 11 µm, 9.5 µm, 7.4 µm, or 6.4 µm. The distance between sub-pixels may be 10 µm or less and may be, specifically, 8 µm, 7.4 µm, or 6.4 µm.

The pixels may have a known arrangement form in a plan view, such as stripe array, delta array, pentile array, or Bayer array. The shape of the sub-pixel in a plan view may be any known shape, and examples thereof include a quadrangle such as a rectangle and a rhombus and a hexagon. If the shape is not an exact shape, but is close to a rectangle, the shape is included in the rectangle. The shape of the sub-pixel and the pixel array can be used in combination.

### [4] Application of organic light-emitting element

The organic light-emitting element according to the present embodiment can be used as a constitutional member of a display apparatus or illuminating apparatus. In addition, examples of the application include an exposure light source of an electrophotographic image formation apparatus, a backlight of a liquid crystal display apparatus, and a light-emitting apparatus having a color filter in a white light source.

The display apparatus may be an image information-processing apparatus that includes an image input unit that inputs image information from an area CCD, a linear CCD, a memory card, or the like and an information processing unit that processes the input information and displays the input image on a display unit. The display apparatus includes a plurality of pixels, and at least one of the plurality of pixels may include the organic light-emitting element of the present embodiment and an active element such as a transistor connected to the organic light-emitting element. On this occasion, the substrate is a semiconductor substrate such as silicon, and the transistor may be MOSFET formed on the substrate. The image display apparatus includes an input unit for inputting image information and a display unit for outputting an image, and the display unit includes the display apparatus of the present embodiment.

The display unit of an image pickup apparatus or inkjet printer may have a touch panel function. The driving method of this touch panel function is not specifically limited and may be an infrared system, an electrostatic capacitance system, a resistive film system, or an electromagnetic induction system. The display apparatus may be used in the display unit of a multifunction printer.

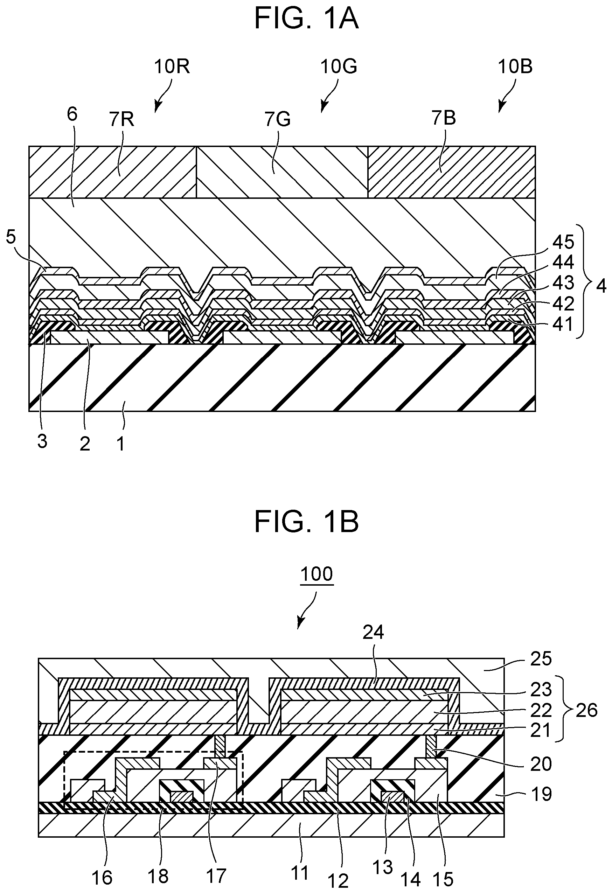

A display apparatus according to the present embodiment will be then described with reference to the drawings. Figs. 1A and 1B are schematic cross-sectional views showing examples of a display apparatus that includes an organic light-emitting element and a transistor being in contact with the organic light-emitting element. The transistor is an example of an active element. The transistor may be a thin-film transistor (TFT).

Fig. 1A is a schematic cross-sectional view of an example of a pixel that is a constitutional element of a display apparatus according to the present embodiment. The pixel includes sub-pixels 10. The sub-pixels are divided into 10R, 10G, and 10B depending on the light emission thereof. The emission color may be distinguished depending on the wavelength of light emitted from the light-emitting layer, or the light emitted from the sub-pixels may be selectively transmitted or color-converted by a color filter of the like.

Each sub-pixel 10 includes a reflective electrode as a first electrode 2 on an interlayer insulation layer 1, an insulation layer 3 covering the ends of the first electrode 2, an organic compound layer 4 covering the first electrode 2 and the insulation layer 3, a transparent electrode as a second electrode 5, a protection layer 6, and a color filter 7.

A transistor and a capacitor element are may be arranged in the lower layer or the inside of the interlayer insulation layer 1.

The transistor and the first electrode 2 may be electrically connected through a contact hole or the like (not shown).

The insulation layer 3 is also called a bank or a pixel separation film and is arranged so as to cover the ends of the first electrode 2 and to surround the first electrode 2. The portion where the insulation layer 3 is not arranged is in contact with the organic compound layer 4 and functions as a light-emitting region.

The organic compound layer 4 includes a hole-injection layer 41, a hole-transporting layer 42, a light-emitting layer 43, a hole-blocking layer 44, and an electron-transporting layer 45.

The second electrode 5 may be a transparent electrode, a reflective electrode, or a semitransparent electrode.

The protection layer 6 prevents moisture from infiltrating into the organic compound layer 4. The protection layer 6 is illustrated as a single layer, but may be a multilayer. Each layer may be an inorganic compound layer or an organic compound layer.

The color filter 7 is divided into 7R, 7G, and 7B depending on the color. The color filter 7 may be formed on a planarizing film (not shown). In addition, a resin protection layer (not shown) may be provided on the color filter 7. The color filter 7 may be formed on a protection layer 6. The color filter 7 provided on an opposite substrate such as a glass substrate may be attached to the protection layer 6.

The display apparatus 100 of Fig. 1B includes an organic light-emitting element 26 and a TFT 18 which is an example of a transistor includes a substrate 11 such as glass or silicon and an insulation layer 12 disposed on the substrate 11. On the insulation layer 12, an active element such as the TFT 18 is arranged, and a gate electrode 13, a gate insulating film 14, and a semiconductor layer 15 of the active element are disposed. The TFT 18 includes a drain electrode 16 and a source electrode 17. An insulating film 19 is disposed above the TFT 18. A positive electrode 21 constituting the organic light-emitting element 26 is connected to the source electrode 17 via a contact hole 20 provided in the insulating film 19.

The electrical connection method between the electrodes (the positive electrode 21 and the negative electrode 23) included in the organic light-emitting element 26 and the electrodes (the source electrode 17 and the drain electrode 16) included in the TFT 18 is not limited to the form shown in Fig. 1B. That is, it is sufficient that one of the positive electrode 21 or the negative electrode 23 is electrically connected to one of the source electrode 17 or the drain electrode 16 of the TFT 18.

The organic compound layer 22 in the display apparatus 100 of Fig. 1B is illustrated as one layer, but the organic compound layer 22 may be a multilayer. On the negative electrode 23, a first production layer 24 and a second protection layer 25 are provided for reducing the degradation of the organic light-emitting element 26.

In the display apparatus 100 of Fig. 1B, a transistor is used as a switching element, but instead of it, another switching element such as an MIM element may be used.

The transistor used in the display apparatus 100 of Fig. 1B is not limited to a thin-film transistor having an active layer on the insulative surface of the substrate and may be a transistor using a single-crystal silicon wafer. Examples of the active layer include single-crystal silicon, amorphous silicon, non-single-crystal silicon such as microcrystal silicon, and a non-single-crystal oxide semiconductor such as an indium zinc oxide and an indium gallium zinc oxide. The thin-film transistor is also called a TFT element.

The transistor included in the display apparatus 100 of Fig. 1B may be formed in a substrate such as a Si substrate. Here, being formed in a substrate means that a transistor is produced by processing a substrate itself, such as a Si substrate. That is, having a transistor in a substrate can also be regarded as that the substrate and the transistor are integrally formed.

In the organic light-emitting element according to the present embodiment, emission luminance is controlled by a TFT which is an example of a switching element, and an image can be displayed through emission luminance of each of a plurality of organic light-emitting elements provided in a plane. The switching element according to the present embodiment is not limited to a TFT and may be a transistor formed of low-temperature polysilicon or an active matrix driver formed on a substrate such as a Si substrate. The term "on the substrate" can also be expressed as "in the substrate". Whether to provide a transistor in the substrate or use a TFT is selected depending on the size of the display unit. For example, if the size is about 0.5 inches, the organic light-emitting element may be provided on a Si substrate.

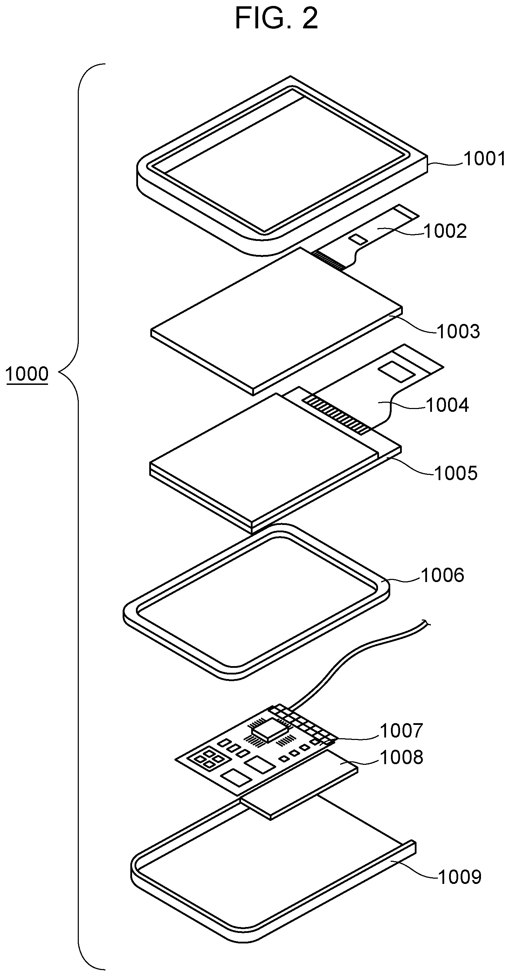

Fig. 2 is a schematic view illustrating an example of the display apparatus according to the present embodiment. The display apparatus 1000 may include a touch panel 1003, a display panel 1005, a frame 1006, a circuit substrate 1007, and a battery 1008 between a top cover 1001 and a bottom cover 1009. The touch panel 1003 and the display panel 1005 are connected to flexible print circuits FPC 1002 and 1004, respectively. On the circuit substrate 1007, a transistor is printed. The battery 1008 does not need to be provided when the display apparatus is a mobile device and may be provided at another position even if the display apparatus is a mobile device.

The display apparatus according to the present embodiment may include color filters having red, green, and blue colors. The red, green, and blue color filters may be arranged in a delta array.

The display apparatus according to the present embodiment may be used in a display unit of a mobile terminal. In such a case, the display apparatus may have both a display function and an operation function. Examples of the mobile terminal include a mobile phone such as a smartphone, a tablet, and a head mount display.

The display apparatus according to the present embodiment may be used in the display unit of an image pickup apparatus that includes an optical unit having a plurality of lenses and an image pickup element that receives light passed through the optical unit. The image pickup apparatus may include a display unit that displays the information acquired by the image pickup element. The display unit may be a display unit exposing to the outside of the image pickup apparatus or a display unit arranged in the finder. The image pickup apparatus may be a digital camera or a digital camcorder.

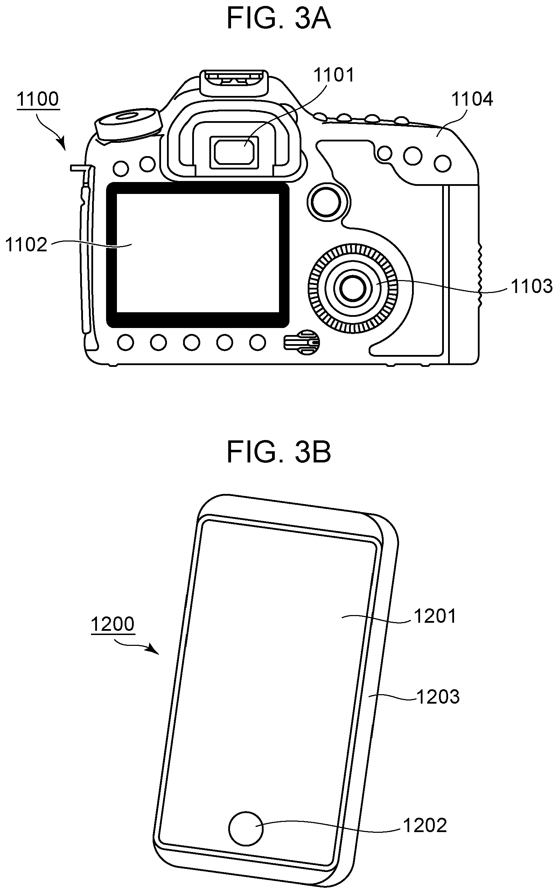

Fig. 3A is a schematic view illustrating an example of the image pickup apparatus according to the present embodiment. The image pickup apparatus 1100 may include a viewfinder 1101, a rear display 1102, an operation unit 1103, and a housing 1104. The viewfinder 1101 may include the display apparatus according to the present embodiment. In such a case, the display apparatus may display not only the images to be captured but also environmental information, image pickup instruction, and so on. The environmental information may be the intensity of external light, the direction of external light, the speed at which a photographic subject moves, the possibility that the subject will be shielded by a shielding material, and so on.

Since the optimum timing for image pickup is a short period of time, the information should be displayed as quickly as possible. Accordingly, a display apparatus using the organic light-emitting element according to the present embodiment may be used, because the organic light-emitting element has a high response speed. The display apparatus using the organic light-emitting element can be used instead of apparatuses that are required to have a high display speed, such as a liquid crystal display apparatus.

The image pickup apparatus 1100 includes an optical unit (not shown). The optical unit includes a plurality of lenses and forms an image on the image pickup element that is accommodated in the housing 1104. The plurality of lenses can adjust the focus point by adjusting the relative positions thereof. This procedure can also be automatically performed. The image pickup apparatus may be called a photoelectric conversion apparatus. The photoelectric conversion apparatus does not sequentially capture images but, instead, can adopt a method of detecting the difference from the previous image, a method of cutting out an image from images that are always being recorded, or the like as the method for capturing images,

Fig. 3B is a schematic view illustrating an example of electronic equipment according to the present embodiment. The electronic equipment 1200 includes a display unit 1201, an operation unit 1202, and a housing 1203. The housing 1203 may include a circuit, a print substrate having the circuit, a battery, and a communication unit. The operation unit 1202 may be a button or a touch panel-type reaction unit. The operation unit 1202 may be biometrics unit that performs recognition of a fingerprint to unlock a lock. The electronic equipment including the communication unit can be also referred to as communication equipment. The electronic equipment 1200 may include a lens and an image pickup element to further have a camera function. An image captured by the camera function is displayed on the display unit 1201. Examples of the electronic equipment 1200 include a smartphone and a laptop personal computer.

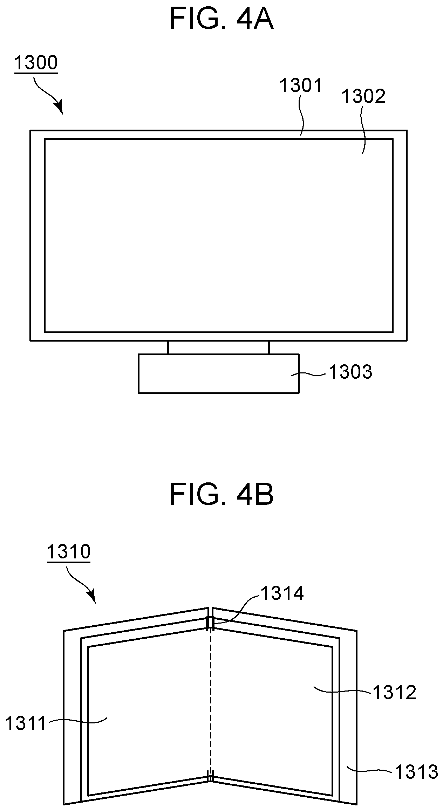

Figs. 4A and 4B are schematic views illustrating examples of a display apparatus according to the present embodiment. Fig. 4A illustrates a display apparatus such as a television monitor and a personal computer (PC) monitor. The display apparatus 1300 includes a frame 1301 and includes a display unit 1302. In the display unit 1302, the light-emitting element according to the present embodiment may be used. The display apparatus 1300 includes a frame 1301 and a base 1303 that supports the display unit 1302. The base 1303 is not limited to the mode shown in Fig. 4A. The lower side of the frame 1301 may also serve as a base. The frame 1301 and the display unit 1302 may be curved. The curvature radius thereof may be 5000 mm or more and 6000 mm or less.

Fig. 4B is a schematic view illustrating another example of the display apparatus according to the present embodiment. The display apparatus 1310 of Fig. 4B is configured to be bendable and is a so-called foldable display apparatus. The display apparatus 1310 includes a first display unit 1311, a second display unit 1312, a housing 1313, and a folding point 1314. The first display unit 1311 and the second display unit 1312 may include the light-emitting element according to the present embodiment. The first display unit 1311 and the second display unit 1312 may be one seamless display apparatus. The first display unit 1311 and the second display unit 1312 can be divided at the folding point. The first display unit 1311 and the second display unit 1312 may display mutually different images, or the first and second display units may display one image.

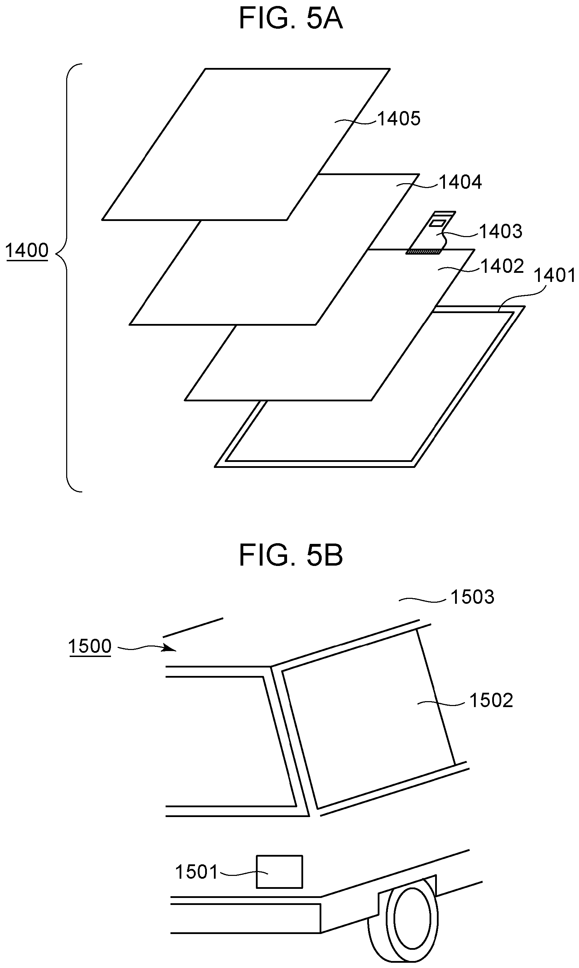

Fig. 5A is a schematic view illustrating an example of the illuminating apparatus according to the present embodiment. The illuminating apparatus 1400 may include a housing 1401, a light source 1402, a circuit substrate 1403, and an optical filter 1404 and a light diffusion unit 1405 through which the light emitted by the light source 1402 transmits. The light source 1402 may include the organic light-emitting element according to the present embodiment. The optical filter 1404 may be a filter to enhance the color rendering properties of a light source. The light diffusion unit 1405 can effectively diffuse the light of the light source, such as lighting up, and can thereby deliver light to a wide range. The optical filter 1404 and the light diffusion unit 1405 may be arranged on an emission side of illumination light. As needed, a cover may be provided at the outermost portion.

The illuminating apparatus is, for example, an apparatus that illuminates the inside of a room. The illuminating apparatus may be an apparatus that emits light in any color of white, daylight white, and other colors from blue to red. The illuminating apparatus may include a light modulating circuit that modulates light in these colors or a toning circuit that tones the emission color. The illuminating apparatus may include the organic light-emitting element of the present embodiment and a power source circuit that is connected to the organic light-emitting element. The power source circuit is a circuit that converts an AC voltage into a DC voltage. The illuminating apparatus may include an inverter circuit. White has a color temperature of 4200 K, and daylight white has a color temperature of 5000 K. The illuminating apparatus may include a color filter.

The illuminating apparatus according to the present embodiment may include a heat release unit. The heat release unit dissipates heat inside the apparatus to the outside, and examples thereof include a metal with a high specific heat and liquid silicon.

Fig. 5B is a schematic view illustrating an automobile as an example of a moving object according to the present embodiment. The automobile includes a tail lamp, which is an example of a lighting device. The automobile 1500 includes a tail lamp 1501, and the tail lamp may be in a form in which the tail lamp is turned on when a braking operation or the like is performed.

The tail lamp 1501 may include the organic light-emitting element according to the present embodiment. The tail lamp 1501 may include a protective member that protects the organic light-emitting element. The material of the protective member is not limited as long as having high strength to some extent and having transparency, and the protective member may be made of polycarbonate. The polycarbonate may be mixed with a furandicarboxylic acid derivative, an acrylonitrile derivative, or the like.

The automobile 1500 may include a body 1503 and a window 1502 attached thereto. The window 1502 may be a transparent display instead of a window for viewing the front and rear of the automobile. The transparent display may include the organic light-emitting element according to the present embodiment.

In such a case, the constituent material, such as an electrode, of the organic light-emitting element is constituted of a transparent member.

The moving object according to the present embodiment may be a ship, an airplane, a drone, or the like. The moving object may include a body and a lighting device mounted on the body. The lighting device may emit light for notifying the position of the body. The lighting device includes the organic light-emitting element according to the present embodiment.

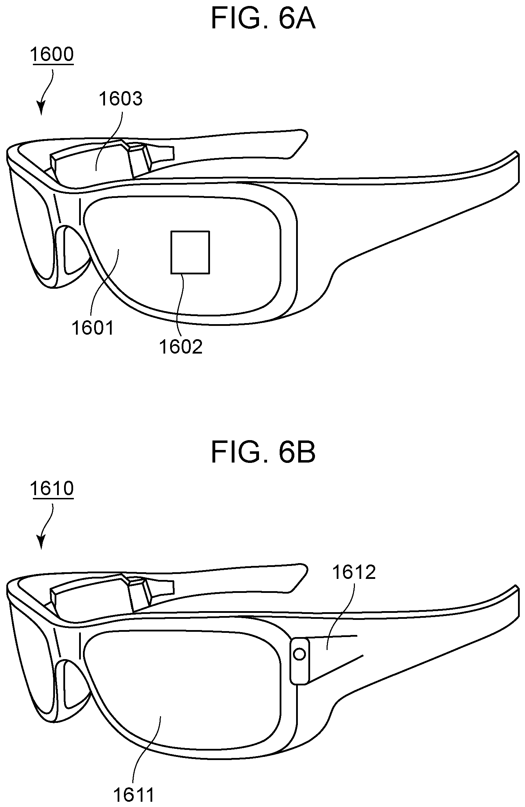

Application examples of the display apparatus of each of the above-described embodiments will be described with reference to Figs. 6A and 6B. The display apparatus can be applied to a system that can be worn as a wearable device such as smart glasses, a head mount display (HMD), and a smart contact lens. The image pickup and display apparatus that is used in such an application example includes an image pickup apparatus capable of photoelectrically converting visible light and a display apparatus capable of emitting visible light.

Fig. 6A is a schematic view illustrating an example of a wearable device according to an embodiment of the present disclosure. Glasses 1600 (smart glasses) according to one application example will be described using Fig. 6A. The image pickup apparatus 1602, such as a CMOS sensor and an SPAD, is provided to the lens 1601 of the glasses 1600 on the front side. The display apparatus of each of the above-described embodiments is provided on the rear side of the lens 1601.

The glasses 1600 further includes a controller 1603. The controller 1603 functions as a power source that supplies power to the image pickup apparatus 1602 and the display apparatus. The controller 1603 controls the operation of the image pickup apparatus 1602 and the display apparatus. An optical system for concentrating light in the image pickup apparatus 1602 is formed in the lens 1601.

Fig. 6B is a schematic view illustrating another example of a wearable device according to an embodiment of the present disclosure. Glasses 1610 (smart glasses) according to one application example will be described using Fig. 6B. The glasses 1610 include a controller 1612, and the controller 1612 is equipped with an image pickup apparatus corresponding to the image pickup apparatus 1602 of Fig. 6A and a light emitting apparatus. In the lens 1611, the image pickup apparatus in the controller 1612 and an optical system for projecting the light emitted from the display apparatus are formed, and an image is projected to the lens 1611. The controller 1612 functions as a power source that supplies power to the image pickup apparatus and the display apparatus and controls the operation of the image pickup apparatus and the display apparatus.

The controller 1612 may include a gaze detection unit for detecting the wearer's gaze. The detection of a gaze may use infrared light. The infrared light-emitting unit emits infrared light toward an eyeball of a user gazing the displayed image. An image pickup unit including a light-receiving element detects reflected light of the infrared light emitted from the eyeball to obtain a pickup image of the eyeball. A decrease in the image quality is reduced by a device for reducing light from the infrared light-emitting unit to the display unit in a plan view. The gaze of a user to the displayed image is detected from the pickup image of the eyeball obtained by capturing infrared light. An any known method can be used for the gaze detection using the pickup image of the eyeball. As an example, a gaze detection method based on the Purkinj e image by reflection of irradiation light on the cornea can be used. More specifically, gaze detection treatment based on a pupil-cornea reflection method is performed. The gaze vector representing the direction (rotation angle) of an eyeball is calculated using the pupil-cornea reflection method based on the image of pupil included in the pickup image of the eyeball and the Purkinje image, and the gaze of the user is detected.

The display apparatus according to one embodiment of the present disclosure includes an image pickup apparatus including a light-receiving element and may control the displayed image on the display apparatus based on the gaze information of the user from the image pickup apparatus. Specifically, the display apparatus determines a first viewing region closely viewed by the user and a second viewing region other than the first viewing region based on the gaze information. The first viewing region and the second viewing region may be determined by the controller of the display apparatus, or may be determined by an external controller and received. In the display region of the display apparatus, the display resolution of the first viewing region may be controlled to be higher than the display resolution of the second viewing region. That is, the resolution of the second viewing region may be lower than the resolution of the first viewing region.

The display region includes a first display region and a second display region different from the first display region, and a high-priority region is determined from the first display region and the second display region based on the gaze information. The first viewing region and the second viewing region may be determined by the controller of the display apparatus, or may be determined by an external controller and received. The resolution of the high-priority region may be controlled to be higher than the resolution of the region other than the high-priority region. That is, the resolution of a relatively low-priority region may be decreased.

The first viewing region and the high-priority region may be determined using AI. The AI may be a model configured to estimate the angle of the gaze from an image of the eyeball and the distance until a target in front of the gaze using the image of the eyeball and the direction actually viewed by the eyeball in the image as teaching data. The AI program may be included in the display apparatus, an image pickup apparatus, or an external apparatus. When the external apparatus includes the AI program, the AI program is transmitted to the display apparatus through communication.

When display is controlled based on visual detection, the display apparatus can be applied to smart glasses further including an image pickup apparatus for capturing the outside. The smart glasses can display the captured external information in real time.

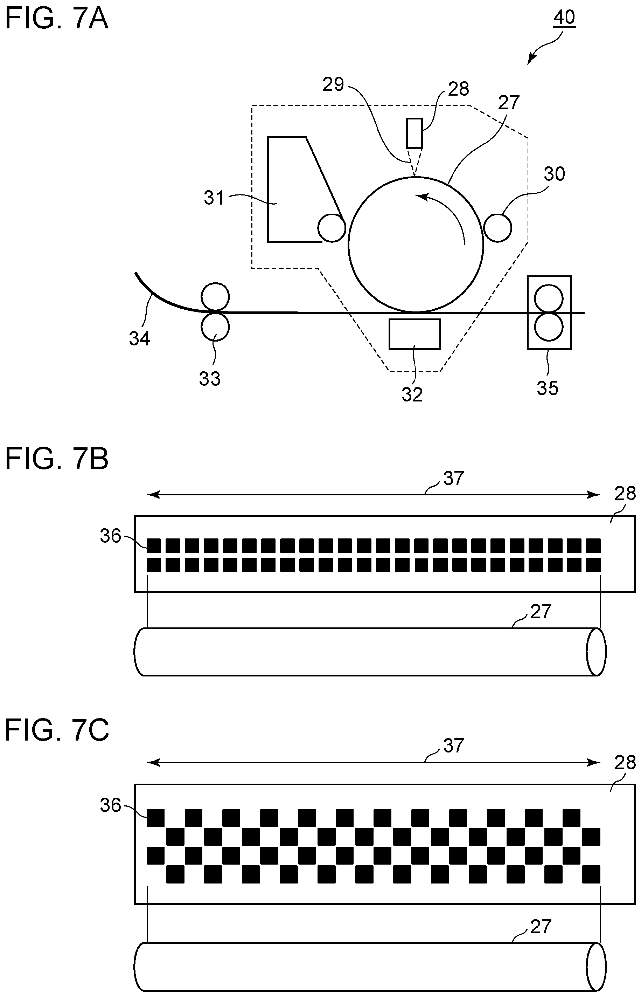

Fig. 7A is a schematic view illustrating an example of an image formation apparatus according to an embodiment of the present disclosure. The image formation apparatus 40 is an electrographic image formation apparatus and includes a photoreceptor 27, an exposure light source 28, a charging portion 30, a development portion 31, a transfer device 32, a transport roller 33, and a fixing device 35. Light 29 is irradiated from the exposure light source 28 to form an electrostatic latent image on the surface of the photoreceptor 27. This exposure light source 28 includes the organic light-emitting element according to the present embodiment. The development portion 31 includes a toner or the like. The charging portion 30 charges the photoreceptor 27. The transfer device 32 transfers the developed image to a recording medium 34. The transport roller 33 transports the recording medium 34. The recording medium 34 is, for example, paper. The fixing device 35 fixes the image formed on the recording medium 34.

Figs. 7B and 7C are diagrams each showing an exposure light source 28 and are schematic views showing the state in which a plurality of light-emitting units 36 are arranged on a long substrate. The arrow 37 represents a direction parallel to the axis of the photoreceptor and represents the column direction in which the organic light-emitting elements are arranged. This column direction is the same as the direction of the axis around which the photoreceptor 27 rotates. This direction can also be called the longitudinal direction of the photoreceptor 27. Fig. 7B shows a configuration in which the light-emitting units 36 are arranged along the longitudinal direction of the photoreceptor 27. Fig. 7C shows a configuration different from that of Fig. 7B and is a configuration in which light-emitting units 36 are arranged alternately in the column direction in each of the first column and the second column. The first column and the second column are arranged in different positions in the row direction. In the first column, a plurality of light-emitting units 36 are arranged with spaces. The second column includes light-emitting units 36 at the positions corresponding to the spaces between the light-emitting units 36 in the first column. That is, a plurality of light-emitting units 36 are arranged with spaces also in the row direction. The arrangement of Fig. 7C can be rephrased as, for example, a lattice arrangement, a hound's-tooth check arrangement, or a checkered pattern.

As described above, display with good image quality and stable for a long time is possible by using an apparatus using the organic light-emitting element according to the present embodiment. In addition, both good visibility and power-saving display in the outdoors by high-efficient and high-luminant light output are possible by using an apparatus using the organic light-emitting element according to the present embodiment.

### Examples

The present disclosure will now be described by Examples. However, the present disclosure is not limited to such Examples.

### Example 1: Synthesis of Exemplary Compound A10

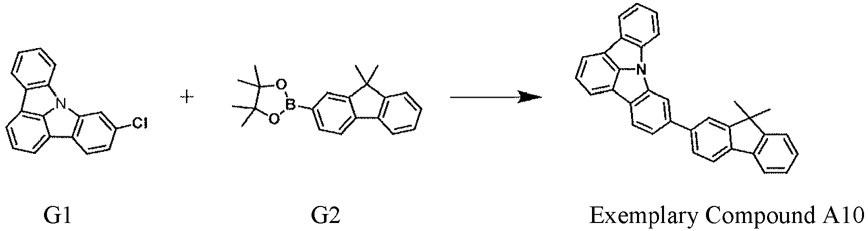

Reagents and solvents shown below were added to a 500-mL eggplant flask, heated to 90°C under a nitrogen flow, and stirred at this temperature (90°C) for 5 hours.
Compound G1: 1.00 g (3.63 mmol);
Compound G2: 1.16 g (3.63 mmol);
Pd(OAc)₂: 25 mg (0.11 mmol);
s-phos: 163 mg (0.36 mmol);
K₃PO₄: 1.54 g (7.26 mmol);
Toluene: 30 mL; and
Water: 1 mL.

After completion of the reaction, methanol was added to the eggplant flask, followed by filtration to obtain a crude product as a filtrate. The crude product was purified by silica gel column chromatography (chlorobenzene) and then recrystallized from xylene to obtain 1.02 g (yield: 65%) of Exemplary Compound A10.

Exemplary Compound A10 was subjected to mass spectrometry using MALDI-TOF-MS (manufactured by Bruker, Autoflex LRF).

### MALDI-TOF-MS

Measured value: m/z = 433
Calculated value: C₃₃H₃₃N = 433

### Examples 2 to 30: Synthesis of Exemplary Compounds

Exemplary Compounds were synthesized as in Example 1 except that the raw material G1 and the raw material G2 in Example 1 were changed to raw material 1 and raw material 2, respectively. The raw material 1 and raw material 2 of each Example are shown in Tables 7 to 9. The measured values (m/z) of the mass spectrometry results measured as in Example 1 are shown in Tables 7 to 9.

**Table 7**

| Example | Exemplary Compound | Raw material 1 | Raw material 2 | m/z |
|---|---|---|---|---|
| 2 | A2 | 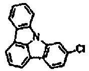 | 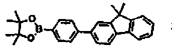 | 509 |
| 3 | A5 | 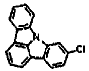 | 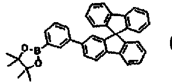 | 331 |
| 4 | A6 | 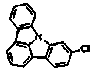 | 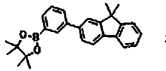 | 509 |
| 5 | A7 | 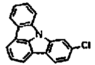 | 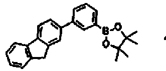 | 481 |
| 6 | A9 | 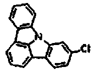 | 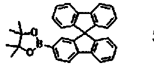 | 555 |
| 7 | A12 | 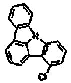 | 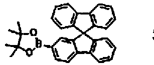 | 555 |
| 8 | A17 | 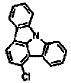 | 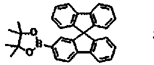 | 555 |
| 9 | B1 | 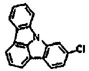 | 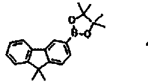 | 433 |
| 10 | B5 | 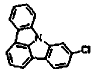 | 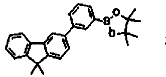 | 509 |
| 11 | B17 | 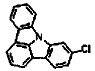 | 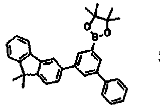 | 585 |

**Table 8**

| Example | Exemplary Compound | Raw material 1 | Raw material 2 | m/z |
|---|---|---|---|---|
| 12 | B24 | 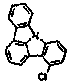 | 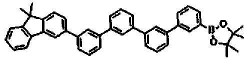 | 737 |
| 13 | B33 | 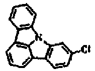 | 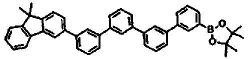 | 661 |
| 14 | B41 | 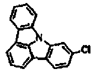 | 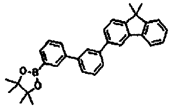 | 585 |
| 15 | B49 | 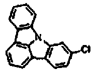 | 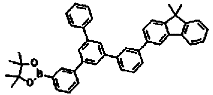 | 737 |
| 16 | C1 | 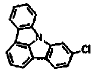 | 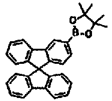 | 555 |
| 17 | C2 | 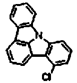 | 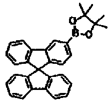 | 555 |
| 18 | C3 | 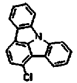 | 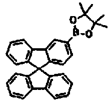 | 555 |
| 19 | C5 | 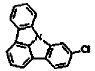 | 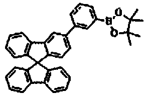 | 631 |
| 20 | C7 | 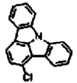 | 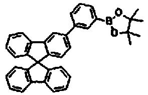 | 631 |
| 21 | C9 | 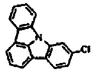 | 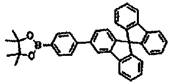 | 631 |

**Table 9**

| Example | Exemplary Compound | Raw material 1 | Raw material 2 | m/z |
|---|---|---|---|---|
| 22 | C13 | 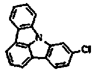 | 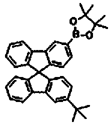 | 611 |
| 23 | C14 | 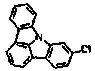 | 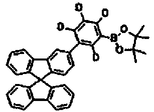 | 635 |
| 24 | C17 | 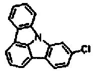 | 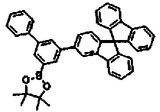 | 707 |
| 25 | C19 | 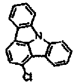 | 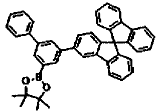 | 707 |
| 26 | C33 | 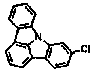 | 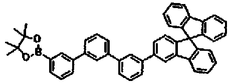 | 783 |
| 27 | C40 | 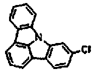 | 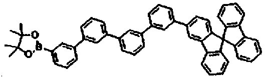 | 859 |
| 28 | C43 | 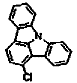 | 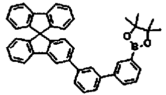 | 707 |
| 29 | C57 | 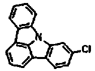 | 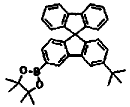 | 611 |
| 30 | C60 | 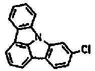 | 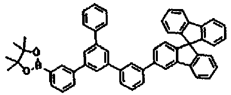 | 859 |

### Example 31

An organic light-emitting element of a bottom-emission-type structure in which a positive electrode, a hole-injection layer, a hole-transporting layer, an electron-blocking layer, a light-emitting layer, a hole-blocking layer, an electron-transporting layer, an electron-injection layer, and a negative electrode were sequentially formed on a substrate was produced.

A film of ITO was formed on a glass substrate and was then patterned to form a desired ITO electrode (positive electrode). On this occasion, the thickness of the ITO electrode was adjusted to 100 nm. The substrate thus-provided with the ITO electrode was used as an ITO substrate in the following processes. Subsequently, vacuum deposition by resistance heating was performed in a vacuum chamber of 1.33 × 10⁻⁴ Pa to sequentially form organic compound layers and electrode layers shown in Table 10 on the ITO substrate. On this occasion, the electrode area of the opposing electrodes (metal electrode layer and negative electrode) was adjusted to 3 mm².

**Table 10**

| | Material | | | Film thickness (nm) |
|---|---|---|---|---|
| Negative electrode | AL | | | 100 |
| Electron injection layer (EIL) | LiF | | | 1 |
| Electron-transporting layer (ETL) | ET2 | | | 20 |
| Hole-blocking layer (HBL) | ET11 | | | 20 |
| Light-emitting layer (EML) | Host | A10 | Mass ratio A10 : GD11 =90 : 10 | 20 |
| | Guest | GD11 | | |
| Electron-blocking layer (EBL) | HT19 | | | 15 |
| Hole-transporting layer (HTL) | HT3 | | | 30 |
| Hole injection layer (HIL) | HT16 | | | 5 |

The element characteristics of the obtained element were measured and evaluated. When the maximum external quantum efficiency in Comparative Example 2 described below was defined as 1.00, the light emission efficiency in this Example was 1.15. Furthermore, a continuous driving test was performed at a current density of 100 mA/cm², and the time at which the luminance degradation rate reached 5% was measured. This time was referred to as an element life. When the time at which the luminance degradation rate in Comparative Example 2 reached 5% was defined as 1.0, the element life in this Example was 1.3.

### Examples 32 to 54 and Comparative Examples 1 to 3

Organic light-emitting elements were produced by the same method as in Example 31 except that the compounds shown in Table 11 were used. The element characteristics of the obtained elements were measured and evaluated as in Example 31. The results of measurement are shown in Table 11.

**Table 11**

| | HIL | HTL | EBL | EML | | HBL | ETL | Light emission efficiency | Element life |
|---|---|---|---|---|---|---|---|---|---|
| | | | | Host | Guest | | | | |
| Example 32 | HT16 | HT3 | HT19 | A2 | GD11 | ET11 | ET2 | 1.10 | 1.1 |
| Example 33 | HT16 | HT3 | HT19 | A5 | GD11 | ET11 | ET2 | 1.20 | 1.3 |
| Example 34 | HT16 | HT3 | HT19 | A6 | GD11 | ET11 | ET2 | 1.15 | 1.2 |
| Example 35 | HT16 | HT3 | HT19 | A7 | GD11 | ET11 | ET2 | 1.10 | 1.1 |
| Example 36 | HT16 | HT3 | HT19 | A9 | GD11 | ET11 | ET2 | 1.20 | 1.4 |
| Example 37 | HT16 | HT3 | HT19 | B1 | GD11 | ET11 | ET2 | 1.25 | 1.4 |
| Example 38 | HT16 | HT3 | HT19 | B5 | GD11 | ET11 | ET2 | 1.20 | 1.3 |
| Example 39 | HT16 | HT3 | HT19 | B17 | GD12 | ET11 | ET2 | 1.25 | 1.3 |
| Example 40 | HT16 | HT3 | HT19 | B24 | GD12 | ET11 | ET2 | 1.20 | 1.2 |
| Example 41 | HT16 | HT3 | HT19 | B33 | GD11 | ET12 | ET2 | 1.10 | 1.4 |
| Example 42 | HT16 | HT3 | HT19 | B41 | GD11 | ET11 | ET2 | 1.20 | 1.3 |
| Example 43 | HT16 | HT3 | HT19 | C1 | GD11 | ET11 | ET2 | 1.30 | 1.6 |
| Example 44 | HT16 | HT3 | HT19 | C5 | GD11 | ET11 | ET2 | 1.25 | 1.5 |
| Example 45 | HT16 | HT3 | HT19 | C7 | GD11 | ET11 | ET2 | 1.20 | 1.3 |
| Example 46 | HT16 | HT3 | HT19 | C9 | GD11 | ET11 | ET2 | 1.20 | 1.3 |
| Example 47 | HT16 | HT3 | HT19 | C13 | GD11 | ET11 | ET2 | 1.30 | 1.6 |
| Example 48 | HT16 | HT3 | HT19 | C17 | GD11 | ET11 | ET2 | 1.25 | 1.5 |
| Example 49 | HT16 | HT3 | HT19 | C19 | GD12 | ET11 | ET2 | 1.25 | 1.3 |
| Example 50 | HT16 | HT3 | HT19 | C33 | GD12 | ET11 | ET2 | 1.30 | 1.5 |
| Example 51 | HT16 | HT3 | HT19 | C40 | GD11 | ET12 | ET2 | 1.15 | 1.5 |
| Example 52 | HT16 | HT3 | HT19 | C43 | GD11 | ET12 | ET2 | 1.10 | 1.4 |
| Example 53 | HT16 | HT3 | HT19 | C57 | GD11 | ET11 | ET2 | 1.30 | 1.6 |
| Example 54 | HT16 | HT3 | HT19 | C60 | GD12 | ET11 | ET2 | 1.30 | 1.5 |
| Comparative Example 1 | HT16 | HT3 | HT19 | Comparative Compound 1-a | GD11 | ET11 | ET2 | 0.95 | 1.0 |
| Comparative Example 2 | HT16 | HT3 | HT19 | Comparative Compound 1-b | GD11 | ET11 | ET2 | 1.00 | 1.0 |
| Comparative Example 3 | HT16 | HT3 | HT19 | Comparative Compound 1-a | GD12 | ET11 | ET2 | 1.00 | 1.0 |

As shown in Table 11, it was demonstrated that the organic light-emitting elements each using an organic compound according to the present embodiment exhibited excellent light emission efficiency and element life compared to organic light-emitting elements using Comparative Compound 1-a or Comparative Compound 1-b. This is inferred that the organic compound according to the present embodiment had a high T1 energy and thereby could efficiently transfer T1 excitons to the light-emitting dopant.

Furthermore, it was demonstrated by Examples 33 and 44 that the organic light-emitting element using Exemplary Compound C5 had more excellent light emission efficiency and element life than the organic light-emitting element using Exemplary Compound A5. This is because that since Exemplary Compound C5 binds to L at R₃₀₃ or R₃₀₆, Exemplary Compound C5 had a high T1 energy, and energy transfer to the light-emitting dopant was more accelerated.

Furthermore, it was demonstrated by Examples 44 and 46 that the organic light-emitting element using Exemplary Compound C5 had more excellent light emission efficiency and element life than the organic light-emitting element using Exemplary Compound C9. This is because that since L possessed by Exemplary Compound C5 is a metaphenylene group, Exemplary Compound C5 had a high T1 energy, and energy transfer to the guest molecule was more accelerated.

Furthermore, it was demonstrated by Examples 38 and 44 that the organic light-emitting element using Exemplary Compound C5 had more excellent light emission efficiency and element life than the organic light-emitting element using Exemplary Compound B5. This is because that since the spirofluorene skeleton possessed by Exemplary Compound C5 is a bulkier structure than the fluorene skeleton possessed by Exemplary Compound B5, the compatibility between Exemplary Compound C5 and the light-emitting dopant was improved, and energy transfer from Exemplary Compound C5 to the guest molecule was accelerated.

Furthermore, it was demonstrated by Examples 44 and 45 that the organic light-emitting element using Exemplary Compound C5 had more excellent light emission efficiency and element life than the organic light-emitting element using Exemplary Compound C7. This is because that since Exemplary Compound C5 binds to L at R₁₀₂ or R₁₀₇, the bond between the indolocarbazole unit and L was less twisted, the T1 energy of Exemplary Compound C5 was increased, and energy transfer from Exemplary Compound C5 to the light-emitting dopant was accelerated.

Furthermore, it was demonstrated by Examples 43 and 44 that the organic light-emitting element using Exemplary Compound C1 had more excellent light emission efficiency and element life than the organic light-emitting element using Exemplary Compound C5. This is because that in Exemplary Compound C1, the interaction between Exemplary Compound C1 molecules can be more suppressed by the direct bond between indolocarbazole and spirofluorene.

As described above, the Exemplary Compounds according to the present embodiment are organic compounds having high T1 energies. An organic light-emitting element exhibiting high light emission efficiency can be obtained by using the organic compound according to the present embodiment in an organic light-emitting element. Furthermore, an organic light-emitting element exhibiting an excellent element life can be obtained by using the organic compound according to the present embodiment in an organic light-emitting element.

The organic compound according to the present disclosure can have the following configurations.

According to the present disclosure, it is possible to provide an organic compound with a high T1 energy.

While the present disclosure has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An organic compound represented by formula (1):
(A)ₙ₁-L-(B)ₙ₂ (1)
in formula (1), (A) has a structure represented by formula (2), and (B) has a structure represented by formula (3) or formula (4): 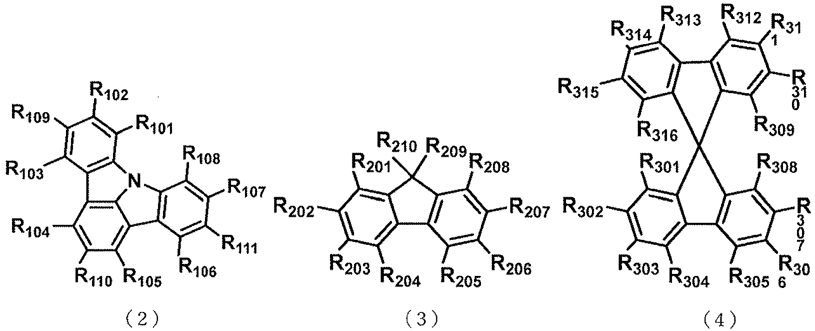
in formula (2), R₁₀₁ to R₁₁₁ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group; and any one of R₁₀₁ to R₁₁₁ is a binding position to L,
in formula (3), R₂₀₁ to R₂₀₈ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group; R₂₀₉ and R₂₁₀ are each independently selected from the group consisting of a hydrogen atom, a deuterium atom, a halogen atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, and a cyano group; and any one of R₂₀₁ to R₂₀₈ is a binding position to L,
in formula (4), R₃₀₁ to R₃₀₈ are each independently selected from the group consisting of a hydrogen atom, a deuterium atom, a halogen atom, a substituted or unsubstituted alkyl group, a substituted or unsubstituted alkoxy group, a substituted or unsubstituted silyl group, a substituted or unsubstituted amino group, and a cyano group; any one of R₃₀₁ to R₃₀₈ is a binding position to L; and R₃₀₉ to R₃₁₆ are each independently selected from the group consisting of a hydrogen atom, a deuterium atom, a halogen atom, a substituted or unsubstituted alkyl group, a substituted or unsubstituted alkoxy group, a substituted or unsubstituted aryl group, a substituted or unsubstituted heterocyclic group, a substituted or unsubstituted aryloxy group, a substituted or unsubstituted heteroaryloxy group, a substituted or unsubstituted silyl group, a substituted or unsubstituted amino group, and a cyano group,
L is a linking group derived from a substituted or unsubstituted phenyl group, a linking group derived from a substituted or unsubstituted naphthyl group, a combination thereof, or a direct bond,
n1 and n2 are each an integer of 1 or more and 3 or less; in a case that n1 is 2 or more, (A)s have the same or different structures; and in a case that n2 is 2 or more, (B)s have the same or different structures, and
in a case where, R₁₀₁ to R₁₁₁ are each independently selected from the group consisting of a hydrogen atom, a deuterium atom, a fluorine atom, an alkyl group having 1 or more and 4 or less carbon atoms, an alkoxy group having 1 or more and 4 or less carbon atoms, and a trimethylsilyl group.

2. The organic compound according to Claim 1, wherein in formula (2), any one of R₁₀₂, R₁₀₃, R₁₀₄, R₁₀₅, R₁₀₆, or R₁₀₇ binds to L, and
in a case where, R₁₀₂ or R₁₀₇ binds to L.

3. The organic compound according to Claim 1or 2, wherein in formula (3) or (4), any one of R₂₀₂, R₂₀₃, R₂₀₄, R₂₀₅, R₂₀₆, or R₂₀₇ binds to L; or any one of R₃₀₂, R₃₀₃, R₃₀₄, R₃₀₅, R₃₀₆, or R₃₀₇ binds to L, and
in a case where, R₂₀₃ or R₂₀₆ binds to L; or R₃₀₃ or R₃₀₆ binds to L.

4. The organic compound according to any one of Claims 1 to 3, wherein in formula (1), (B) is a structure represented by formula (4).

5. The organic compound according to any one of Claims 1 to 4, wherein formula (1) is any one of formulae (5) to (8): 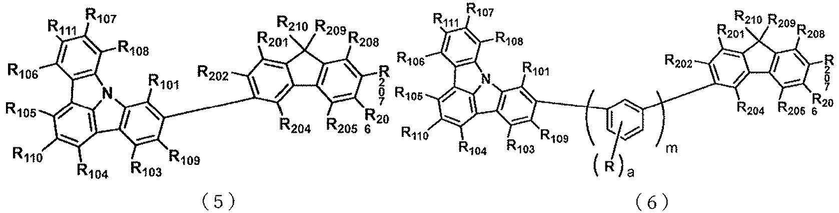 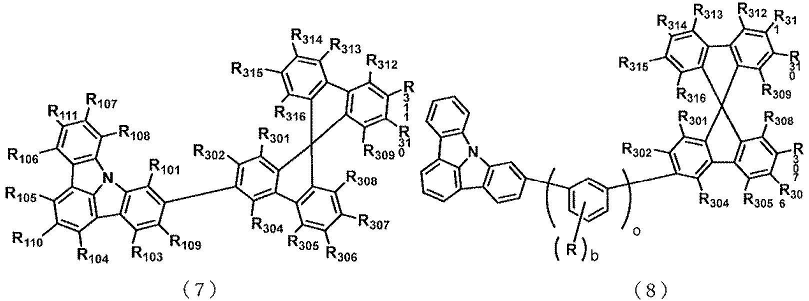
in formula (6), R is a deuterium atom, a substituted or unsubstituted alkyl group, a substituted or unsubstituted alkoxy group, a substituted or unsubstituted aryl group, a substituted or unsubstituted heterocyclic group,
a substituted or unsubstituted aryloxy group, a substituted or unsubstituted heteroaryloxy group, a substituted or unsubstituted silyl group, a substituted or unsubstituted amino group, or a cyano group; a is an integer of 0 or more and 4 or less, m is an integer of 1 or more and 4 or less; and when there is a plurality of Rs, Rs are the same or different, and
in formula (7), R is a deuterium atom, a substituted or unsubstituted alkyl group, a substituted or unsubstituted alkoxy group, a substituted or unsubstituted aryl group, a substituted or unsubstituted heterocyclic group,
a substituted or unsubstituted aryloxy group, a substituted or unsubstituted heteroaryloxy group, a substituted or unsubstituted silyl group, a substituted or unsubstituted amino group, or a cyano group; b is an integer of 0 or more and 4 or less; o is an integer of 1 or more and 4 or less; and in a case there is a plurality of Rs, Rs are the same or different.

6. The organic compound according to Claim 5, wherein formula (1) is formula (7).

7. The organic compound according to any one of Claims 1 to 6, wherein in formulae (3) and (4), R₂₀₁ to R₂₀₈ are each independently selected from the group consisting of a hydrogen atom, a deuterium atom, a fluorine atom, an alkyl group having 1 or more and 6 or less carbon atoms, an alkoxy group having 1 or more and 4 or less carbon atoms, and a trimethylsilyl group; R₂₀₉ and R₂₁₀ are each independently selected from the group consisting of a hydrogen atom, a deuterium atom, an alkyl group having 1 or more and 4 or less carbon atoms, and an aryl group having 6 or more and 12 or less carbon atoms; and R₃₀₁ to R₃₁₆ are each independently selected from the group consisting of a hydrogen atom, a deuterium atom, a fluorine atom, an alkyl group having 1 or more and 6 or less carbon atoms, an alkoxy group having 1 or more and 4 or less carbon atoms, and a trimethylsilyl group, and
in a case where, R₂₀₁ to R₂₀₈ are hydrogen atoms, R₂₀₉ and R₂₁₀ are methyl groups, and R₃₀₁ to R₃₁₆ are each independently selected from the group consisting of a hydrogen atom and an alkyl group having 1 or more and 4 or less carbon atoms.

8. An organic light-emitting element (26) comprising:
a first electrode;
a second electrode; and
an organic compound layer (22) disposed between the first electrode and the second electrode, wherein
at least one layer of the organic compound layer (22) includes the organic compound according to any one of Claims 1 to 7.

9. The organic light-emitting element (26) according to Claim 8, wherein the organic compound layer (22) includes a light-emitting layer (43), and the light-emitting layer (43) includes the organic compound,
in a case where, the light-emitting layer (43) further includes a light-emitting material,
the organic compound has a lowest excited triplet energy that is higher than that of the light-emitting material, and
in a case where, the light-emitting material emits phosphorescence.

10. A display apparatus (1000) comprising:
a plurality of pixels, wherein
at least one of the pixels includes the organic light-emitting element (26) according to Claim 8 or 9 and a transistor connected to the organic light-emitting element (26).

11. A photoelectric conversion apparatus (1100) comprising:
an image pickup element that receives light; and
a display unit (1102) that displays an image captured by the image pickup element, wherein
the display unit (1102) includes the organic light-emitting element (26) according to Claim 8 or 9.

12. An image display apparatus (1300, 1310) comprising:
a display unit (1302, 1311, 1312) that includes the organic light-emitting element (26) according to Claim 8 or 9; and
a housing (1301, 1313) in which the display unit (1302, 1311, 1312) is provided.

13. Electronic equipment (1200) comprising:
a display unit (1201) that includes the organic light-emitting element (26) according to Claim 8 or 9;
a housing (1203) in which the display unit (1201) is provided; and
a communication unit that is provided in the housing (1203) and communicates with the outside.

14. A wearable device (1600, 1610) comprising:
a display unit that includes the organic light-emitting element (26) according to Claim 8 or 9;
an optical system for concentrating light of the display unit; and
a controller (1603, 1612) for controlling the display of the display unit.

15. A moving object (1500) comprising:
a lighting device (1501) including the organic light-emitting element (26) according to Claim 8 or 9; and
a body (1503) in which the lighting device (1501) is provided.
